# EUROPEAN PATENT APPLICATION

(11) **EP 4 782 035 A2**
(43) Date of publication of application: **29.07.2026**
(21) Application number: 26185494.7
(22) Date of filing: 21.11.2018
(51) Int. Cl.: A61M 11/04

(54) **ELECTRONIC VAPORIZER SESSIONING**

(30) Priority: 22.11.2017 US 201762590142 P
(62) Divisional of application: 18819484.9
(71) Applicant: Juul Labs, Inc., Washington DC 20004 (US)
(72) Inventor: Bowen, Adam, Washington DC, 20004 (US); Cheung, Brandon, Washington, DC, 20004 (US); Monsees, James, Washington, 20004 (US); Wacyk, Roxolana, Washington DC, 20004 (US)
(74) Representative: Thum, Bernhard

(57) **Abstract**

Devices, systems and methods for electronic vaporizer sessioning are presented. Usage preferences of a preferred usage of the vaporizable material by a user of a vaporizer device are received via a user interface of an application or the vaporizer device. Usage data representing the usage of the vaporizable device are also received by the application. The application compares the usage data with the usage preferences within a predetermined time period, and determines a set of operational settings for the vaporizer device that correspond to the user's preferred usage of the vaporizable material. The operational settings are transmitted to the vaporizer device over a wireless channel for controlling an operation of the vaporizer device.

## Description

### CROSS-REFERENCE TO RELATED APPLICATIONS

This application claims priority to U.S. Provisional Patent Application No. 62/590,142, filed November 22, 2017, which is entirely incorporated herein by reference.

### TECHNICAL FIELD

The apparatuses, devices, systems, and methods described herein relate to vaporizing devices, such as electronic vaporizer devices, and to methods of using, controlling, making, such devices. The apparatuses, devices, systems, and methods may optionally be configured to provide or include providing information to a user indicating an amount of vapor consumed over a certain time period.

### BACKGROUND

Vaporizing devices, which can also be referred to as electronic vaporizer devices or e-vaporizer devices, can be used for delivery of vapor containing one or more active ingredients by inhalation of the vapor by a user of the vaporizing device. Electronic vaporizer devices are gaining increasing popularity both for prescriptive medical use, in delivering medicaments, and for consumption of tobacco and other plant-based smokeable materials. Electronic vaporizer devices in particular may be portable, self-contained and convenient for use. Typically, such devices are controlled by one or more switches, buttons or the like (controls) on the vaporizer, although a number of devices that may wirelessly communicate with an external controller (e.g., smartphone) have recently become available.

Such wireless control has been primarily limited to temperature setting and other features that were already, and perhaps more conveniently, performed on the device itself. These systems may not automate or calibrate the operation of the device based on detection of the material or type of material loaded into the device. Such systems also may not typically track dosage or vaporizer "sessioning" information and/or allow modification of the device based on dosing or sessioning information. Further, currently described systems may not provide social interaction with other users.

For example with regard to dosing, previous attempts to determine the dosage of vapor and/or an active ingredient in the vapor have been unsatisfactory. Systems that pre-determine dosage by restricting the amount of material to be delivered in a session assume, often incorrectly, that all of the material will be inhaled, and may not be adjustable for partial dosages. Such systems may also meter the amount of material, and require accurate measurement of the mass and/or volume of material being delivered for vaporization, or measure the difference between a starting mass/volume and post-delivery mass or volume. These measurements may be difficult, requiring a high level of accuracy and expense, and may result in inaccurate results. Further, current dose or session controlling electronic smoking devices typically control the dose delivered without a link to or actual knowledge of the actual clinical and medical needs of the user, and may not allow a controlled dose or session to be adjusted based on the user biometrics such as weight, age, symptoms, etc. Existing systems may also lack features that allow a user to customize usage based on their habits and goals, as well as their social needs.

The systems, apparatuses, devices, and methods described herein address at least these problems and concerns.

### SUMMARY

This document describes devices, systems and methods for electronic vaporizer sessioning. In some aspects, a vaporizer system includes a vaporizer device having one or more sensors for sensing a usage of a vaporizable material in a period of time. The one or more sensors are configured to generate usage data representing the usage of the vaporizable material. The vaporizer device further includes a first transceiver for communicating the usage data to a wireless channel. The system further includes a mobile communication device having a second transceiver configured for being in communication with the first transceiver of the vaporizer device via the wireless channel. The mobile communication device includes a memory that stores an application, a processor for executing the application, and a user interface for displaying an output of the application.

In yet other aspects a vaporizer device is disclosed that is configured to communicate with an application being executed by a mobile computing device. The application is configured to receive usage preferences of preferred usage of a vaporizable material by a user of the vaporizer device, and further configured to determine a set of operational settings for the vaporizer device that correspond to the user's preferred usage of the vaporizable material. In some implementations, the vaporizer device includes a vaporizer body having a power source, a receptacle, and a controller. The vaporizer device further includes a cartridge configured for mating with the receptacle of the vaporizer body, and having a reservoir to hold the vaporizable material and a heater for vaporizing the vaporizable material based on a usage of the vaporizer device by the user, the heater being responsive to the set of operational settings managed by the controller. The vaporizer device further includes one or more sensors associated with the vaporizer body and/or the cartridge for sensing the usage of a vaporizable material in a period of time, the one or more sensors being configured to generate usage data representing the usage of the vaporizable material. The vaporizer device further includes a transceiver for communicating the usage data to the mobile computing device via a wireless channel, and for receiving the set of operational settings to control the vaporizer device according to the usage preferences of the preferred usage of the vaporizable material received from the user of the vaporizable device.

In yet other aspects, methods of operating a vaporizer device that contains a vaporizable material for being vaporized by a user are presented. The vaporizer device is configured to communicate with an application that is executed by a mobile computing device having a user interface. In some implementations, a method includes the steps of receiving, by the application, usage preferences of a preferred usage of the vaporizable material by the user of the vaporizer device, and receiving, by the application from one or more sensors associated with the vaporizer device, usage data representing a usage of the vaporizable device. The method includes the steps of comparing, by the application, the usage data with the usage preferences within a predetermined time period, and determining, by the application, a set of operational settings for the vaporizer device that correspond to the user's preferred usage of the vaporizable material. The method further includes the step of transmitting, by the mobile computing device to the vaporizer device over a wireless channel, the set of operational settings for the vaporizer device to control an operation of the vaporizer device.

In accordance with some implementations of the system, the application is configured to receive, via the user interface, usage preferences of a preferred usage of the vaporizable material by a user of the vaporizer device. The application is further configured to receive, via the second transceiver, the usage data representing the usage of the vaporizable device, and to compare the usage data with the usage preferences within a predetermined time period. The application is further configured to determine a set of operational settings for the vaporizer device that correspond to the user's preferred usage of the vaporizable material, and to transmit, via the second transceiver to the vaporizer device over the wireless channel, the set of operational settings for the vaporizer device.

The details of one or more variations of the subject matter described herein are set forth in the accompanying drawings and the description below. Other features and advantages of the subject matter described herein will be apparent from the description and drawings, and from the claims. While certain features of the currently disclosed subject matter are described for illustrative purposes in relation to electronic vaporizer devices, it should be readily understood that such features are not intended to be limiting. The claims that follow this disclosure are intended to define the scope of the protected subject matter.

### BRIEF DESCRIPTION OF THE DRAWINGS

The accompanying drawings, which are incorporated in and constitute a part of this specification, show certain aspects of the subject matter disclosed herein and, together with the description, help explain some of the principles associated with the disclosed implementations. In the drawings:
FIG. 1 illustrates a block diagram of a vaporizer, in accordance with some example implementations;
FIG. 2A illustrates an example exploded view of the vaporizer of FIG. 1, in accordance with some example implementations;
FIG. 2B illustrates another example view of the vaporizer of FIG. 1, in accordance with some example implementations;
FIG. 2C illustrates another example view of the vaporizer of FIG. 1, in accordance with some example implementations;
FIG. 2D illustrates an example view of the vaporizer cartridge of FIG. 1, in accordance with some example implementations;
FIG. 3 illustrates a bock diagram of another vaporizer, in accordance with some example implementations;
FIG. 4A illustrates an example view of the vaporizer of FIG. 3, in accordance with some example implementations;
FIG. 4B illustrates another example view of the vaporizer of FIG. 3, in accordance with some example implementations;
FIG. 5 illustrates a block diagram of communication among a vaporizer, a user device, and a server, in accordance with some example implementations;
FIG. 6 illustrates an example device which may be used to implement one or more of the described features and/or components, in accordance with some example implementations; and
FIG. 7 illustrates a flowchart of an example method of providing sessioning information to a user, in accordance with some example implementations.

When practical, similar reference numbers denote similar structures, features, or elements.

### DETAILED DESCRIPTION

Implementations of the current subject matter includes methods, devices, apparatuses, articles of manufacture, and systems relating to vaporizing and/or aerosolizing one or more materials for inhalation by a user. Example implementations include vaporizer devices and systems including vaporizer devices. The term "vaporizer" is used generically in the following description and claims to refer to any of a self-contained apparatus, an apparatus that includes two or more separable parts (e.g., a vaporizer body that includes a battery and/or other hardware, and a cartridge that includes and/or is configured to hold a vaporizable material), and/or the like. A "vaporizer system" as used herein may include one or more components, such as a device in communication (e.g., wirelessly or over a wired connection) with a vaporizer and optionally also the vaporizer itself. A vaporizer or one or more components of a vaporizer system consistent with implementations of the current subject matter may be configured for user control and operation. As used herein, an "aerosol" may refer to "vapor" and/or a "vaporizer."

Examples of vaporizers consistent with implementations of the current subject matter include electronic vaporizers, electronic cigarettes, e-cigarettes, or the like. In general, such vaporizers are hand-held devices that heat (by convection, conduction, radiation, or some combination thereof) a vaporizable material to provide an inhalable dose of the material. The vaporizable material used with a vaporizer may be provided within a cartridge (e.g., a part of the vaporizer that contains the vaporizable material in a reservoir or other container and that can be refillable when empty or disposable in favor a new cartridge containing additional vaporizable material of a same or different type. A vaporizer may be a cartridge-using vaporizer, a cartridge-less vaporizer, or a multi-use vaporizer capable of use with or without a cartridge. For example, a multi-use vaporizer may include a heating chamber (e.g., an oven) configured to receive a vaporizable material directly in the heating chamber and also to receive a cartridge having a reservoir or the like for holding the vaporizable material. In various implementations, a vaporizer may be configured for use with liquid vaporizable material (e.g., a carrier solution in which an active and/or inactive ingredient(s) are suspended or held in solution or a liquid form of the vaporizable material itself) or a solid vaporizable material. A solid vaporizable material may include a plant material that emits some part of the plant material as the vaporizable material (e.g., such that some part of the plant material remains as waste after the vaporizable material is emitted for inhalation by a user) or optionally can be a solid form of the vaporizable material itself such that all of the solid material can eventually be vaporized for inhalation. A liquid vaporizable material can likewise be capable of being completely vaporized or can include some part of the liquid material that remains after all of the material suitable for inhalation has been consumed.

Consistent with some implementations of the current subject matter, a vaporizer and/or vaporizer system may be configured to identify a vaporizable material to be vaporized, and to adjust the operation of the vaporizer accordingly. For example, a vaporizer may be adapted to receive a cartridge or other pre-loaded container holding a vaporizable material (e.g., the vaporizable material a solution of nicotine, cannabis, and/or another active ingredient) and to identify and/or determine information about the vaporizable material and/or the cartridge or other pre-loaded container, such as one or more of: a type of vaporizable material, a concentration of vaporizable material in a solution or other non-pure form of a vaporizable material that is contained in a reservoir or other container of the cartridge, an amount (e.g., a mass, volume, etc.) of vaporizable material in a reservoir or other container of the cartridge, a configuration of the cartridge (e.g., what specific components or types of components such as a heater power or configuration, one or more electrical properties, etc. are present in the cartridge), a lot number of the cartridge, a date of manufacture of the cartridge, an expiration date after which the cartridge should not be used, a manufacture or fill date for the cartridge, or the like.

A vaporizer consistent with implementations of the current subject matter may be configured to connect (e.g., wirelessly connect or over a wired connection) to a communication device (or optionally devices) in communication with the vaporizer. Such a device can be a component of a vaporizer system as discussed above, and can include first communication hardware, which can establish a wireless communication channel with second communication hardware of the vaporizer. For example, a device used as part of a vaporizer system may include a general purpose computing device (e.g., a smartphone, a tablet, a personal computer, some other portable device such as a smartwatch, or the like) that executes software to produce a user interface for enabling a user of the device to interact with a vaporizer. In other implementations of the current subject matter, such a device used as part of a vaporizer system can be a dedicated piece of hardware such as a remote control or other wireless or wired device having one or more physical or soft (e.g., configurable on a screen or other display device and selectable via user interaction with a touch-sensitive screen or some other input device like a mouse, pointer, trackball, cursor buttons, or the like) interface controls. Although the term "device" is used herein, the term "apparatus" is intended.

A device that is part of a vaporizer system as defined above can be used for any of one or more functions, such as controlling dosing (e.g., dose monitoring, dose setting, dose limiting, user tracking, etc.), controlling sessioning (e.g., session monitoring, session setting, session limiting, user tracking, etc.), obtaining locational information (e.g., location of other users, retailer/commercial venue locations, vaping locations, relative or absolute location of the vaporizer itself, etc.), vaporizer personalization (e.g., naming the vaporizer, locking/password protecting the vaporizer, adjusting one or more parental controls, associating the vaporizer with a user group, registering the vaporizer with a manufacturer or warranty maintenance organization, etc.), engaging in social activities (e.g., games, social media communications, interacting with one or more groups, etc.) with other users, or the like. The terms "sessioning", "session", "vaporizer session," or "vapor session," is used generically in the following description and claims to refer to a period devoted to the use of the vaporizer. The period can include a time period, number of doses, amount of vaporizable material, and/or the like.

In some implementations of the current subject matter, a vaporizer can include functionality for communicating with a cartridge containing a vaporizable material. The vaporizer may also be in communication with a device that is part of a vaporizer system, although this is not required. The vaporizer, whether under control of or otherwise in communication with a device that is part of a vaporizer system or as a standalone unit separate from a vaporizer system can be configured such that operation of the vaporizer can be modified, controlled, etc. based on one or more parameters that are received from the cartridge or are accessed from a database or other information source based on the identification of the cartridge.

For example, a vaporizer consistent with implementations of the current subject matter can be configured to recognize a cartridge and recite (and in some cases transmit) or otherwise acquire information about the cartridge. In other words, a computing element such as a controller or the like that is associated with a vaporizer body can obtain information about the cartridge via some form of data exchange. A variety of methods of cartridge recognition by a vaporizer are within the scope of the current subject matter, including those described in more detail below. Any of the approaches described herein may be performed with or without the addition of wireless communication/connectivity also described herein, although such wireless connectivity as described herein may be advantageously applied, as will be described in greater detail below.

Implementations of the current subject matter also include methods of using a vaporizer and/or a vaporizer system for functions such as determining and/or controlling a dose, amount, or the like of one or more chemical species of the vaporizable material or of the vaporizable material itself.

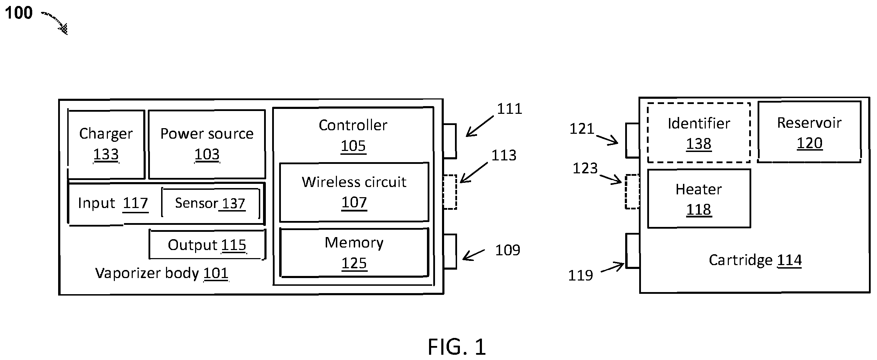

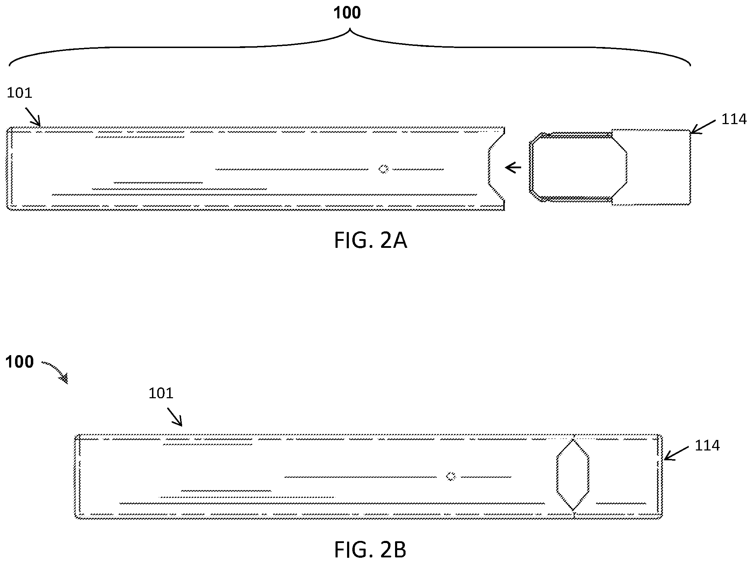

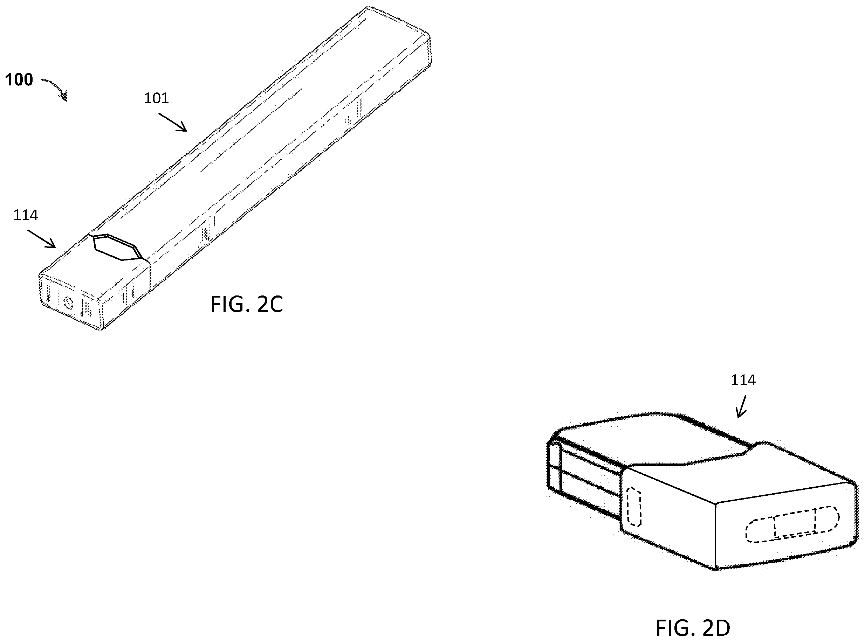

FIG. 1 illustrates a block diagram of a vaporizer 100, in accordance with some example implementations. FIG. 2A illustrates an example exploded view of the vaporizer 100 of FIG. 1, in accordance with some example implementations. As illustrated, the vaporizer 100 can include the vaporizer body 101 and the cartridge 114 separated from the vaporizer body 101. FIG. 2B illustrates an example (front plan) view of the vaporizer 100 of FIG. 1, in accordance with some example implementations. In some aspects, the vaporizer 100 illustrated in FIG. 2A, when assembled, can look like the vaporizer 100 illustrated in FIG. 2B. FIG. 2C illustrates an example (side perspective) view of the vaporizer 100 of FIG. 1, in accordance with some example implementations. In some aspects, the vaporizer 100 illustrated in FIG. 2A, when assembled, can look like the vaporizer 100 illustrated in FIG. 2C. FIG. 2D illustrates an example view of the vaporizer cartridge 114 of FIG. 1, in accordance with some example implementations. In some implementations, the cartridge 114 can hold and/or be configured to hold a liquid vaporizable material. For example, when a vaporizer includes a cartridge (such as the cartridge 114), the cartridge 114 may include one or more reservoirs 120 of and/or for vaporizable material. Any appropriate vaporizable material may be contained within the reservoir 120 of the cartridge 114, including solutions of nicotine or other organic materials.

As illustrated, the vaporizer 100 of in FIG. 1 includes a vaporizer body 101. The vaporizer body 101 may include a housing enclosing a power source 103 (e.g., a device or system that stores electrical energy for on-demand use), which may be a battery, capacitor, a combination thereof, or the like, and which may be rechargeable or non-rechargeable. The housing may also enclose a controller 105, which may include a processor. In the examples shown, a cartridge 114 may be attached on, in, or partially in the vaporizer body 101.

A processor of the controller 105 may include circuitry to control operation of a heater 118, which can optionally include one or more heating elements for vaporizing a vaporizable material contained within the cartridge 114, for example within a reservoir or container that is part of the cartridge 114. In various implementations, the heater 118 may be present in the vaporizer body 101 or within the cartridge 114 (as shown in FIG. 1), or both. The controller circuitry may include one or more clocks (oscillators), charging circuitry, I/O controllers, memory, etc. Alternatively or in addition, the controller circuitry may include circuitry for one or more wireless communication modes, including Bluetooth, near-field communication (NFC), WiFi, ultrasound, ZigBee, RFID, etc. The vaporizer body 101 may also include a memory 125 that may be part of the controller 105 or otherwise in data communication with the controller. The memory 125 may include volatile (e.g., random access memory) and/or non-volatile (e.g., read-only memory, flash memory, solid state storage, a hard drive, other magnetic storage, etc.) memory or data storage.

Further with reference to FIG. 1, a vaporizer 100 may include a charger 133 (and charging circuitry which may be controlled by the controller 105), optionally including an inductive charger and/or a plug-in charger. For example, a universal serial bus (USB) connection may be used to charge the vaporizer 100 and/or to allow communication over a wired connection between a computing device and the controller 105. The charger 133 may charge the onboard power source 103. A vaporizer 100 consistent with implementations of the current subject matter may also include one or more inputs 117, such as buttons, dials, or the like, and/or sensors 137, including accelerometers or other motion sensors, capacitive sensors, flow sensors, or the like. These sensors 137 may be used by the vaporizer 100 to detect user handling and interaction. For example, detection of a rapid movement (such as a shaking motion) of the vaporizer 100 may be interpreted by the controller 105 (e.g., through receipt of a signal from one or more of the sensors 137) as a user command to begin communication with a user device that is part of a vaporizer system and that can be used for controlling one or more operations and/or parameters of the vaporizer 100 as described in more detail below. Additionally or alternatively, detection of a rapid movement (such as a shaking motion) of the vaporizer 100 may be interpreted by the controller 105 (e.g., through receipt of a signal from one or more of the sensors 137) as a user command to cycle through a plurality of temperature settings to which the vaporizable material held within the cartridge 114 is to be heated by action of the heater 118. In some optional variations, detection of removal of the cartridge 114 by the controller 105 (e.g., through receipt of a signal from one or more of the sensors 137) during a cycling-through of the plurality of temperature settings may act to establish the temperature (e.g., when the cycle is at a desired temperature, a user may remove the cartridge 114 to set the desired temperature). The cartridge 114 may then be re-engaged with the vaporizer body 101 by the user to allow use of the vaporizer 100 with the heater controlled by the controller 105 consistent with the selected temperature setting. The plurality of temperature settings may be indicated through one or more indicators on the vaporizer body 101.

A vaporizer consistent with implementations of the current subject matter may also include one or more outputs 115. Outputs 115 as used herein can refer to any of optical (e.g., LEDs, displays, etc.), tactile (e.g., vibrational, etc.), or sonic (e.g., piezoelectric, etc.) feedback components, or the like, or some combination thereof.

A vaporizer 100 consistent with implementations of the current subject that includes a cartridge 114 may include one or more electrical contacts (such as the electrical contacts 109, 111, 113 shown in FIG. 1) on or within the vaporizer body 101 that may engage complementary contacts 119, 121, 123 (e.g., pins or receptacles) on the cartridge 114 when the cartridge is engaged with the vaporizer body 101. The contacts on the vaporizer body are generally referred to as "vaporizer body contacts" and those on the cartridge are generally referred to as "cartridge contacts." These contacts may be used to provide energy from the power source 103 to the heater 118 in implementations of the current subject matter in which the heater 118 is included in the cartridge 114. For example, when the cartridge contacts and the vaporizer body contacts are respectively engaged by coupling of the cartridge 114 with the vaporizer body 101, an electrical power circuit can be formed allowing control of power flow from the power source 103 in the vaporizer body 101 to the heater 118 in the cartridge 114. A controller 105 in the vaporizer body 101 can regulate this power flow to control a temperature at which the heater 118 heats a vaporizable material contained in the cartridge 114.

Any appropriate electrical contact may be used, including pins (e.g., pogo pins), plates, and the like. In addition, as described below, in some implementations of the current subject matter one-way or two-way communication is provided between the vaporizer body 101 and the cartridge 114 through one or more electrical contacts, which may include the electrical contacts used to provide energy from the power source 103 to the heater 118. The cartridge 114 and the vaporizer body 101 may be removably coupled together, e.g., by engaging a portion of a housing of the cartridge 114 with the vaporizer body 101 and/or the vaporizer housing in a mechanical connection (e.g., a snap and/or friction fit) or the like. Alternatively or additionally, the cartridge 114 and the vaporizer body 101 may be coupled magnetically or via some other coupling or engaging mechanism.

Any of the cartridges described herein may include one or more identifiers 138. The identifier 138 may be recognized, detected, and/or read by the vaporizer body 101, and may convey information about the vaporizable material contained within the cartridge and/or about the cartridge 114 itself. The identifier 138 may include a readable and/or readable/writable cartridge memory. The identifier 138 may include circuitry for receiving and/or transmitting information between the cartridge 114 and the vaporizer body 101. For example, a data exchange circuit may include the cartridge memory, which stores information (e.g., data characterizing one or more parameters of the cartridge), and additional circuitry that forms a data exchange circuit in cooperation with other circuitry on a vaporizer body 101 when the cartridge 114 is coupled to the vaporizer body 101.

In some implementations of the current subject matter, the identifier 138 is passive and may include codes or markings (e.g., bar codes, quick response (QR) codes, etc.). In some examples, the identifier 138 may be structural (e.g., one or more pins, projections, etc.) on the cartridge 114 that may be detected by the vaporizer body 101. Visual or mechanical identifiers may be identified directly by the vaporizer body 101 using an imaging device (e.g., camera, etc.) or reading device (e.g., optical reading) integrated into the vaporizer body (not shown in FIG. 1), or via communication through a separate device, such as a smartphone. For example, a user may take an image of the identifier 138 (e.g., code, marking, etc.) and transmit the code or information derived from the code (such as the information about the vaporizable material and/or the cartridge) to the vaporizer body 101 via wireless circuitry 107, or optionally over a wired connection. A wireless connection (e.g., a wireless communication channel) can be established between first communication hardware of the device and second communication hardware of the vaporizer. The first and second communication hardware can respectively include transceivers for use with one or more wireless communication protocols, non-limiting examples of which are described below.

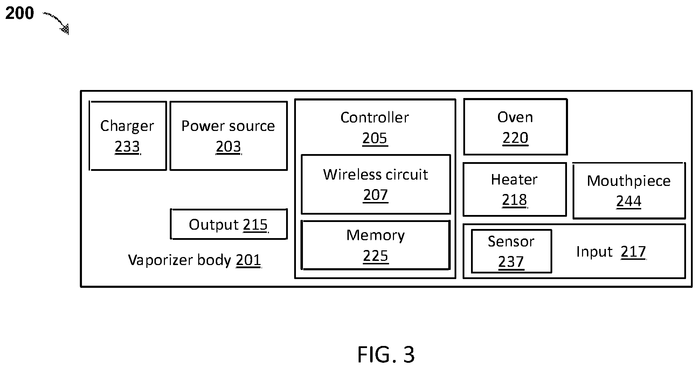

FIG. 3 shows a schematic diagram of a vaporizer 200 that does not use a cartridge (but may still optionally accept a cartridge), but may instead use a loose-leaf material. The vaporizer 200 in FIG. 3 may include loose vaporizable material that may be placed in an oven 220 (e.g., vaporization chamber). Many of the same elements present in the vaporizer 100 using cartridge 114 shown in FIG. 1 and FIGs. 2A-D may also be included as part of a vaporizer 200 that does not use cartridges. For example, a cartridge-free vaporizer 200 may include a vaporizer body 201 with control circuitry 205 which may include power control circuitry, and/or wireless circuitry 207, and/or memory 225. A power source 203 (e.g., battery, capacitor, etc.) may be charged by a charger 233 (and may include charging control circuitry, not shown). The vaporizer 200 may also include one or more outputs 215 and one or more inputs 217 with sensors 237. In addition, the vaporizer 200 may include one or more heaters 218 that heat an oven 220 or other heating chamber. The heater 218 may be controlled using the resistance of the heater 218 to determine the temperature of the heater, e.g., by using the temperature coefficient of resistivity for the heater. A mouthpiece 244 may also be included.

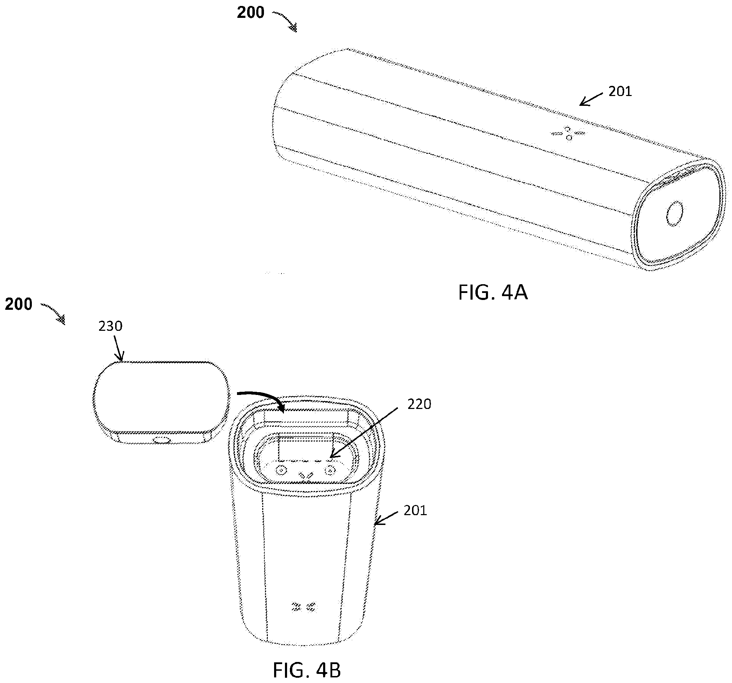

FIG. 4A shows a side perspective of an exemplary vaporizer device 200 with a vaporizer body 201. In the bottom perspective view of FIG. 4B, a lid 230 is shown removed from the vaporizer body 201, exposing the oven/vaporization chamber 220.

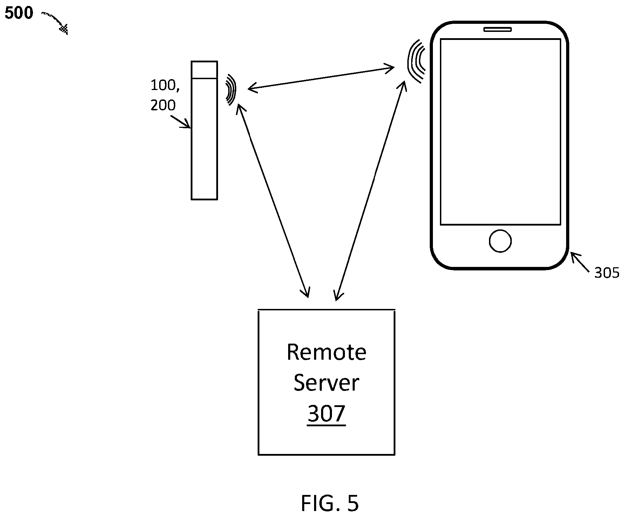

FIG. 5 shows a schematic representation of communication among a vaporizer 100, 200, a digital device 305 that wirelessly communicates with the vaporizer 100, 200 and a remote server 307 that may communicate directly with the vaporizer 100, 200, or through the digital device 305. The digital device 305 may be a hand-held mobile device such as a smartphone, smartwatch, tablet, etc., or a desktop or laptop computing device. As noted above, the digital device 305 may optionally be a dedicated remote control device.

In general, as illustrated schematically in FIG. 5, any of the vaporizer apparatuses described herein (such as the vaporizer 100, 200) may remotely communicate with a remote server 307 and/or a digital device 305 such as a wearable electronics device (e.g., Google Glass, smartwatch, smartwear, etc.) and/or a smartphone, smartwatch, etc. Thus, any of these vaporizers 100, 200 may include a communications interface (wireless circuitry 107, 207) that may be implemented through a communication chip (e.g., second communication hardware) in or on the vaporizer 100, 200. Exemplary wireless chips may include, but are not limited to, a Bluetooth chip, such as Parani BCD 210 or Texas Instruments (TI) CC2650 Bluetooth Single-Chip Solution, an NFC-enabled chip (such as Qualcomm's QCA1990), that allows for NFC communication, or enhanced Wi-Fi or Bluetooth communication where NFC is used for link setup. As will be described in detail below, one or more of these wireless circuits may be used for communication with or between the cartridge 114 in implementations that are configured for reading a cartridge 114 as schematically shown in FIG. 1. For example, NFC may be used to read an identifier 138 (as RFID tag) on the cartridge 114.

A wireless communication chip may include a Wi-Fi-enabled chip, such as TI's SimpleLink family's CC3000, that can hook the apparatus to Wi-Fi networks. In some implementations, the wireless circuit comprises a subscriber identity module (SIM) card on board of the vaporizer, a Nano-SIM card, or the like (e.g., allowing 3G/4G cellular network communication). Alternative forms of communication may be used to establish two-way communication between a vaporizer 100, 200 and a user device 305.

Connection between the vaporizer 100, 200 and the user device 305 may be automatic (after an initial set-up) or may be initiated by the user through various settings or may be initiated by shaking the vaporizer 100, 200.

As mentioned above, any of the vaporizer apparatuses described herein that include a cartridge may be configured to recognize and/or identify the cartridge. One or more recognition/identification approaches may be used. The vaporizer may determine information about the cartridge and/or the vaporizable material held in the cartridge, such as one or more of: the type of vaporizable material (e.g., nicotine, etc.), the concentration of vaporizable material, the amount of vaporizable material, the configuration of the cartridge (e.g., heater, electrical properties, etc.), the lot number of the cartridge, the date of manufacture of the cartridge, expiration date, etc. This information may be directly encoded on the cartridge or a reference indicator may be provided that the vaporizer (or a processor in communication with the vaporizer) may use as an index to look up some or all of this information, or a combination of reference number and directly encoded material may be provided.

In some implementations of the current subject matter, the cartridge may be recognized and/or identified by the engagement between the cartridge and the vaporizer. The cartridge may be configured to include a keyed interaction with the vaporizer. For example, the shape of cartridge may be detected by the vaporizer. For example, the cartridge may include n pins or protrusions. These pins can be detected by the vaporizer when the cartridge is inserted (e.g., by completing an electrical connection); for n pins, there are 2*ⁿ* possible combinations of markings.

The cartridge may be configured or identified based on an electrical property that the vaporizer can detect based on an electrical connection with the cartridge. For example, the vaporizer may make electrical contact through two or more electrical contacts with the heater and/or additional electrical contacts and may detect a characteristic resistance, inductance, or time response (e.g., time constant, RC time constant, LC circuit resonance, etc.).

In some implementations of the current subject matter, the cartridge may be recognized and/or identified by markings on the cartridge identified by the vaporizer. These markings may be visible or not visible to a user. For example, the cartridge may be marked with a characteristic UV, IR or other wavelength-specific ink that can be detected by the vaporizer, which may include, e.g., an emitter/detector pair specific to the marker(s). For example, markings may include an infrared-scannable barcode located on the cartridge. In some implementations, the markings may be a pattern, such as a QR code, bar code, etc., that indicate information about the cartridge and/or the contents (vaporizable material) of the cartridge. The markings may be symbolic, including alphanumeric. The markings may be 'read' or detected directly by the vaporizer, which may include a camera or other optical detector, or it may be indirectly detected via communication with a second device (e.g., wearable, smartphone, etc.) having a camera or the like. For example, markings on the cartridge may be detected by a smartphone such as the user device 305; the smartphone may identify the marking using an application (e.g., software) on the smartphone to look up one or more properties from a look-up table, or it may directly communicate the marking to the vaporizer that may look up the properties, and/or it may communicate with a remote server that may look up the properties and communicate them to the vaporizer directly or through the smartphone.

In some implementations of the current subject matter, the cartridge may be recognized by RFID (Radio-Frequency identification) technology. RFID markers have been used in a wide array of applications for inventory control. Some RFID technologies use active devices which contain their own power source and others use passive RFID devices that interact with another powered device that causes the transfer of data without reliance on power at the passive device. For example, a cartridge may include one or more RFID chips or components that can be detected and read by a reader on the vaporizer to identify and receive information about the cartridge.

In some implementations of the current subject matter, the cartridge may be recognized and/or identified by communicating with a memory (e.g., EEPROM) on the cartridge through an electrical connection with the vaporizer. In implementations in which the heater is present on the cartridge, such as the exemplary vaporizer shown in FIG. 1, it may be advantageous to use one or more of the electrical connections on the cartridge (e.g., contacts 119, 121, 123) that are also used to power and/or control the heater to communicate with the memory. This may be particularly challenging where the cartridge may engage with the vaporizer in more than one orientation, and/or where the heater is controlled through this same contact, and modulation of the applied/received electrical signals between the cartridge and the vaporizer may modify the control and/or temperature determination of the heater. One or more additional electrical contacts may be used in addition to those controlling the heater. In general, communication between the cartridge and the vaporizer may be one way (e.g., reading information about the cartridge and/or the vaporizable material from the cartridge by the vaporizer) or it may be two-way (e.g., reading information about the cartridge and/or the vaporizable material and writing information about the operation of the device, e.g., number of uses, duration of use, temperature settings, etc.). Information may be written to the cartridge, and this information may be used to derive other information about the cartridge, including the amount of material left in the cartridge, etc.

In general, any of the vaporizers described herein may estimate, measure and/or predict the amount of vapor and/or material (including active ingredients) in the vapor that can be delivered to a user. For example, as described in detail below, the apparatuses described herein may be used to determine and/or control dosing of the vaporizable material. For example, the current subject matter includes vaporizers and methods of using such vaporizers for accurate and controlled dose delivery of an active ingredient in a vaporizable material (e.g., nicotine, and any other active ingredient/drug) based on user specified, medical, switching or cessation needs. Dose control may include display of dosing information per use, per session (e.g., multiple uses within a predetermined time period, such as 1-15 minutes, 1-30 min, within 1-60 min, 1-90 min, 1-120 min, etc.), per day, or other predetermined and/or user-defined time period. Dose control may also include monitoring dosing (e.g., amount of one or more active ingredient delivered by the apparatus). Dosing control may also or alternatively include controlling the operation of the vaporizer based on the amount of one or more active ingredient delivered by the apparatus over time, including alerting a user when a predetermined (user defined, factory-set, or third-party set) amount or threshold is approached (e.g., within 50%, 75%, 80%, 85%, 90%, 95%, 98%, 99%, etc. of the predetermined amount) or exceeded, and/or stopping (locking, disabling, etc.) operation of the apparatus when the predetermined threshold is met or exceeded. Apparatuses that include dosing (dose) control may include internal logic (circuitry and/or programming, including application-specific integrated circuit (ASIC) logic) for controlling dosing and/or may communicate with an external processor (via a wireless communication link) that performs all or some of the dose control.

Information about the cartridge and/or a vaporizable material held in the cartridge may be particularly helpful in determining dose. For example information such as one or more of: the type of vaporizable material (e.g., nicotine, etc.), the concentration of vaporizable material, the content of the vaporizable material, the amount of vaporizable material, the configuration of the cartridge (e.g., heater, electrical properties, etc.), the lot number of the cartridge, the date of manufacture of the cartridge, expiration date, the thermal properties of the vaporizable material, etc. may be used to accurately estimate dose. In some implementations of the current subject matter, dose and/or use information may be stored (written) on the cartridge (e.g., in a memory).

Vaporizers, vaporizer systems, and methods of using them for user-customization of device settings and drug usage based on activity patterns are also within the scope of the current subject matter. A vaporizers and/or vaporizer system consistent with the current description may allow a user to personalize a vaporizer and engage in social activities.

A vaporizer and/or vaporizer system consistent with implementations of the current subject matter may be configured to facilitate social interaction through the vaporizer. For example, a vaporizer may be configured to share usage information with others, such as third parties, e.g., health care providers, including doctors, etc. for better prescription and administration of medical treatment. A vaporizer and/or vaporizer system may also be configured to communicate with non-medical third parties (e.g., friends, colleagues, etc.), and with unknown third parties (making some or all information publically available). In some implementations, the vaporizers described herein, either by each vaporizer device alone or in communication with one or more communications devices that are part of a vaporizer system, may identify and provide information about the operation, status or user input from the vaporizer to a public or private network. In some implementations of the current subject matter, a vaporizer and/or vaporizer system may be configured to provide one or more interactive games for use by the user and/or multiple users of different (or the same) vaporizers, including multi-player games that may be used with multiple different vaporizers. Games may be tied to the operation of the vaporizer and/or a user's manipulation of the vaporizer (e.g., based on accelerometer output, touch or lip sensing, draw detection, etc.).

A vaporizer and/or vaporizer system consistent with implementations of the current subject matter may also be configured to provide location information, possibly including one or more of information about user location in proximity to one or more of: other users (known or unknown users, specified or unspecified users, etc.), retailers, specific locations (lounges, clubs, vaporizer-friendly locations), etc. A vaporizer and/or vaporizer system may also be configured to facilitate the placing of orders based on use or operation of the vaporizer and/or vaporizer system.

A vaporizer may include a GPS capability or may access GPS information from another device in communication with the vaporizer as part of a vaporizer system.

As will be described herein in greater detail, a vaporizer may be connected to (e.g., in communication with) an additional (e.g., portable, wearable, smartphone, desktop, laptop, etc.) device, which may enable user programmable dose control, real-time usage monitoring, personalized use settings, device lockout and social features. For example, a vaporizer and/or vaporizer system may include features relating to security controls, including parental control, user age control/restriction and anti-theft control. A vaporizer and/or vaporizer system may include anti-theft and/or authentication functions that may lock or otherwise restrict use/operation of the device when stolen and/or when used with counterfeit parts, and may also be configured to allow locking (e.g., parental-lock) for child-proofing, or otherwise preventing unauthorized third party operation. An anti-counterfeiting or other lock-out feature of this type may be implemented using cartridge identifiers. For example, cartridge identifiers from a verified source or supplier can include a hash or some other verification code as part of the identifier, and the vaporizer may lock out use of the vaporizer if a cartridge lacking the necessary hash or verification code is coupled to a vaporizer body. Such a feature can be used to require that a user identity verification is entered at the device in communication with the vaporizer to cause the device to unlock use of the vaporizer. In one example, a cartridge may include an identifier that indicates that it contains a controlled substance and a user may be required by the application on the device (in response to determining this about the cartridge via identifier information received from the cartridge) to verify his or her identity (e.g., via a password entry, a biometric identity verification, etc.) and for the application to verify that the identified user is authorized for use of the controlled substance prior to being able to use the vaporizer with that cartridge coupled to the vaporizer body. In another example, a nicotine-containing cartridge may require user identity verification such that the application on the device only allows use of the vaporizer if a user identity is verified and the user has been registered as being above the minimum age.

In some examples, a security control may be incorporated via an application executing on a device in communication with a vaporizer. For example, an application executing on a device in communication with a vaporizer can receive an identifier of the vaporizer itself or alternatively/additionally of the cartridge and may, based on or otherwise using the identifier, determine whether a security setting is included in a user profile or other settings associated with the vaporizer or cartridge. Consistent with implementations of the current subject matter, such functionality may be entirely or partially included within the vaporizer (and/or cartridge) or they may be distributed between the vaporizer and a user interface that may be presented on an additional device that is part of a vaporizer system, such as a wearable and/or handheld device, laptop, desktop, etc., operating control logic. Control logic or other software functionality for providing these features may include a user interface, and may provide input/output and analysis capability for modulating operation of the vaporizer. Non-limiting options for the first communication hardware of the device and/or the second communication hardware of the vaporizer are described above.

**CARTRIDGE RECOGNITION.** In general, a vaporizer may include one or more techniques for cartridge recognition and/or communication, including the use of a marker (e.g., QR code, IR or US marker, etc.), mechanical and/or electronic keying, or the like. In particular described herein are methods and apparatuses for electronic cartridge recognition and communication, in which the cartridge may electronically communicate, via one-way or in some implementations two-way (including duplex or multiplex) transmission of information, between a cartridge and the vaporizer so that information may be received by the vaporizer from the cartridge. This information may include information about the vaporizable material and/or the cartridge, such as one or more of: type of vaporizable material, concentration of vaporizable material, amount of vaporizable material, volume of the vaporizable material, properties of the vaporizable material (e.g., thermal properties, composition, etc.), configuration of the cartridge (e.g., heater, electrical properties, etc.), lot number, date of manufacture, expiration date, identity verification for the cartridge, and the like.

A cartridge including an identification circuit (also referred to herein as a cartridge identification circuit) may be configured to communicate and transfer such information from the cartridge to the vaporizer. The cartridge identification circuit may include a memory (e.g., an EEPROM). In cartridge variations in which the heater (e.g., a resistive heating element such as a resistive coil or wire) is controlled by the application of energy onto one or more (e.g., 2, 3, 4, etc.) heater electrical contacts that communicate with corresponding contacts on the vaporizer, the cartridge identification circuit may communicate with the vaporizer through the same heater electrical contacts, despite the increased complexity and potential for disruption of the heater.

The cartridge identification circuit may also be configured so that the cartridge may be inserted into the vaporizer in multiple orientations without disrupting the cartridge identification circuit operation.

The same cartridge identity circuit may also be written with information about the cartridge, vaporizable material, and history of the cartridge, including, for example: the usage time and/or total energy applied, etc.

Information stored on the memory (read and/or written) may be encoded, including the use of encryption, error-correction encoding (e.g., hamming code, etc.), or the like. In operation, when the cartridge is first inserted into the vaporizer body, the vaporizer microcontroller may be configured to first determine if a signal can be read off of the cartridge encoding information about the cartridge and/or identifying the cartridge as compatible with the vaporizer. Information may be read using the measurement circuit of the vaporizer. In some implementations, even when a cartridge may not be read (e.g., may not include a cartridge identity circuit or is unable to read from the cartridge identity circuit) the vaporizer may use a default setting.

During operation, the vaporizer may periodically (e.g., after each puff, etc.) write to the memory in the cartridge identity circuit, if detected. The vaporizer may signal to the memory to request a read from the memory similar to how the device writes to memory, and may then disconnect the battery voltage applied to the heater contacts to allow the memory (e.g., EEPROM) to take control of the I/O line and use it to output data, providing a digital output (switching the I/O line low/high) transmitting an output that the vaporizer detects through the resistance measurement circuit. Typically, if the memory is transmitting, it may affect the absolute accuracy of the temperature control; the vaporizer may be configured so that the device does not heat when the memory is transmitting (outputting) and normal heating operation may not trigger the memory into transmitting data.

As will be described in greater detail below, the information stored in the memory of a cartridge identity circuit such as those described herein may be useful for dose control (e.g., calculating and storing dosing information), as well as for security, communications and storage of operational parameters, particularly in devices including a wireless capability. However, cartridge identification may be useful even in the absence of wireless communication capabilities.

As discussed, the memory (e.g., an EEPROM) may store information about the vaporizable material and/or the cartridge. One example of the information that may be stored may include values related to the specific properties of the heating element, such as the nominal heater R (resistance) for the cartridge, including the heating element of the cartridge. This value may be determined and stored at the factory, at the time the device is manufactured/produced, and/or it may be done later. Storing a specific R value for each cartridge in the memory affiliated with that cartridge may be useful for the accurate temperature control for the device, including determining baseline resistance at ambient temperature, as described above. Although resistance/baseline measurement on the manufacturing line may be slightly different from the measurement the device gets for use, a baseline adjustment (determined by algorithm) may also be used. Alternatively or additionally, once a reliable baseline for a cartridge has been determined, this baseline may be related (e.g., in a remote database, on a remote server, etc.) to an ID affiliated with the specific cartridge, so that if the cartridge is removed and reinserted, the same baseline value can also be used (as soon as the cartridge ID is confirmed) which could be a faster check than waiting for stable baseline to be detected.

In general, storing a cartridge characteristic such as the resistance of the heater in the cartridge itself may be also useful for confirming that the connection between the vaporizer and the cartridge is good, and that the vaporizer's resistance measurement circuit is working normally. Thus, in any of the methods and apparatuses described herein, a nominal cartridge resistance may be stored in the cartridge's memory (or may be stored on a remote server/device and retrieved based on a unique cartridge ID) and may be used to confirm that the connection between the device and pod is good, and/or that the device's resistance measurement circuit is working normally, and/or that the cartridge's resistance has not changed since the cartridge was assembled or filled.

As mentioned above, in some implementations, the vaporizer may write usage information to the cartridge's memory; usage information can be used to estimate the amount of vaporizable material that has been removed from the cartridge and the amount of vaporizable material remaining. Usage information may include number of puffs/draws, the dosage delivered, or the like.

**APPLICATION/CONNECTIVITY.** A vaporizer and/or vaporizer system may include software, firmware or hardware that is separate or separable from the vaporizer and that wirelessly communicates with the vaporizer. For example, applications ("apps") may be executed on a processor of a portable and/or wearable device, including smartphones, smartwatches, and the like, which may be referred to as a personal digital device or optionally just a device (e.g., user device 305 in FIG. 3) that is part of a vaporizer system. These digital devices may provide an interface for the user to engage and interact with functions related to the vaporizer, including communication of data to and from the vaporizer to the digital device or the like and/or additional third party processor (e.g., servers such as the remote server 307 in FIG. 3). For example, a user may control some aspects of the vaporizer (temperature, dosage, etc.) and/or data transmission and data receiving to and from vaporizer, optionally over a wireless communication channel between first communication hardware of the device and second communication hardware of the vaporizer. Data may be communicated in response to one or more actions of the user (e.g., including interactions with a user interface displayed on the device), and/or as a background operation such that the user does not have to initiate or authorize the data communication process.

User interfaces may be deployed on a digital device and may aid the user in operating the vaporizer. For example, the user interface operating on a digital device may include icons and text elements that may inform the user of various ways that vaporizer settings can be adjusted or configured by the user. In this manner (or in others consistent with the current subject matter) information about a vaporizer can be presented using a user interface displayed by the communication device. Icons and/or text elements may be provided to allow a user to see information about vaporizer status, such as battery information (charge remaining, vapor draws remaining, time to charge, charging, etc.), cartridge status (e.g., type of cartridge and vaporizable material, fill status of cartridge, etc.), and similar device status. Icons and/or text elements may be provided to allow a user to update internal software (a.k.a., firmware) in the vaporizer. Icons and text elements may be provided to allow a user to set security and/or authorization features of vaporizer, such as setting a PIN code to activate the device or the use of personal biometric information as a means of authentication. Icons and text elements may be provided to allow a user to configure foreground data sharing and related settings.

A vaporizer may include or incorporate one or more authentication features. For example, the user interface ("app") may include, for example, PIN-based authentication, biometric authentication (which can include fingerprint based authentication, iris scan based authentication, facial recognition based authentication, and/or the like). Authorization may include age-analysis, such as an estimation or calculation of user age based on analysis of facial features. Authorization may be used to lock/unlock the vaporizer.

The authentication process can be embodied as a feature of an application that is installed and running on a personal digital device capable of communicating data through the use of wired or wireless methods (e.g., as part of a vaporizer system as described herein). The personal digital device (e.g., smartphone) may have an operating system capable of running application(s).

A vaporizer may be rendered inactive after a period of inactivity, for example by entering into a "sleep mode" when there is no usage detected for a predetermined and/or preset period of time. In some implementations of the current subject matter, in order for the vaporizer to be activated, and thereby be capable of being used by the user for the purpose of generating vapor, the user must be authenticated to ensure that the device is being utilized by the intended end user, and to prevent unauthorized use, or accidental or unintended activation of the device, or use of the device by an individual not of legal age to ingest the active component, including nicotine. Personal identification number (PIN) based authentication may apply a user selected PIN code to authenticate the end use. Biometric authentication may be used, optionally using one or more approaches. For example, a fingerprint based authentication process may authenticate the end user. An iris scan based authentication process may use an eye or iris scan, or the like, to authenticate the end user. Facial recognition based authentication may use a face scan or image processing algorithm to authenticate the end user. Iris scan based authentication and facial recognition based authentication may be particularly useful if the personal digital device has a camera, such as a forward facing camera.

A personal vaporizer may be deactivated following a threshold criteria being met. For example, the vaporizer may be rendered inactive after a period of inactivity. The period of inactivity may be preset and/or selected by the user (e.g., using the control software of running on the personal digital device). Thus, the period of inactivity may be a configurable parameter of the vaporizer. The application software/firmware may include functionality to unlock or activate the vaporizer using authentication, as mentioned above.

An authentication process may be performed. If the authentication process is unsuccessful, the vaporizer may remain deactivated. If the authentication process is successful, the vaporizer may be unlocked and made ready for use.

A vaporizer may perform onboard data gathering, data analysis, and/or data transmission methods. As mentioned, a vaporizer having wired or wireless communication capability may interface with digital consumer technology products such as smart phones, tablet computers, laptop/netbook/desktop computers, wearable wireless technologies such as "smart watches," and other wearable technology such as Google "Glass," or similar through the use of programming, software, firmware, GUI, wireless communication, wired communication, and/or software commonly referred to as application(s) or "apps." A wired communication connection can be used to interface the vaporizer to digital consumer technology products for the purpose of the transmission and exchange of data to/from the vaporizer from/to the digital consumer technology products (and thereby also interfacing with apps running on the digital consumer technology products.) A wireless communication connection can be used to interface the vaporizer to digital consumer technology products for the transmission and exchange of data to/from the vaporizer from/to the digital wireless interface. The vaporizer may use a wireless interface that includes one or more of an infrared (IR) transmitter, a Bluetooth interface, an 802.11 specified interface, and/or communications with a cellular telephone network in order to communicate with consumer technology.

A vaporizer can interface (e.g., communicate) with digital consumer technology products and with apps as a way of relaying information and data to add additional functionality. This additional functionality may include (but is not limited to): (a) setting and/or specifying a desired number of activation cycles over a period of time; (b) setting and/or specifying one or more reminders, alarms, or similar to notifications for a user; (c) setting and/or specifying a user-desired dose Or doses for delivery of active substance(s) per inhalation; (d) setting and/or specifying a desired total delivered dose active substance(s) over a period of time-such as a total daily dose; (e) setting and/or specifying one or more power settings of the vaporizer to modulate a vapor and/or aerosol strength, a vapor and/or aerosol density, a vapor and/or aerosol volume, a vapor and/or aerosol flavor, a vapor and/or aerosol temperature, and/or other vapor and/or aerosol characteristics of a vapor and/or aerosol generated by the vaporizer; (f) setting and/or specifying power settings of the vaporizer to modulate, adjust, configure or similar the settings of the device as they relate to battery life and/or performance; (g) setting and/or specifying configurations of the vaporizer related to the liquid components and formulation; (h) setting and/or specifying ambient temperature based environmental configurations; (i) setting and/or specifying humidity based environmental configurations; (j) setting and/or specifying altitude based environmental configurations; (k) setting and/or specifying temporal (e.g., time) based configurations; (I) setting and/or specifying parameters to minimize, maximize, and/or modulate the functional effects of the taste and/or flavor component of the vapor product; (m) setting and/or specifying functional effect parameters to minimize or maximize the functional effects related to pharmacodynamics and pharmacokinetics of an active ingredient or drug component of the vapor or aerosol product; (n) receiving and/or providing to a user, vaporizer alerts and notifications; (o) receiving and/or providing to a user, vaporizer alerts and notifications related to recharging (e.g., whether a battery (e.g., power source 103 in FIG. 1) needs to be recharged); (p) receiving and/or providing to a user, vaporizer alerts and notifications related to charge status (e.g., whether a battery is fully or partially charged); (q) receiving and/or providing to a user, vaporizer alerts and notifications related to liquid cartridge usage status-such as a number of usages or inhalations taken from a cartridge; (r) receiving and/or providing to a user, vaporizer alerts and notifications related to liquid cartridge remaining status-such as a number of usages or inhalations remaining in a cartridge; (s) receiving and/or providing to a user, alerts and notifications related to time-based liquid cartridge usage status-such as number of usages or inhalations taken over a preset and/or predetermined period of time, for example number of usages or inhalations taken per day; (t) receiving and/or providing to a user, alerts and notifications related to liquid cartridge contents-such as active component(s), strength, dosage (or similar), flavor profile (or similar), and general formulation (or similar); (u) receiving and/or providing to a user, alerts and notifications related to liquid cartridge, liquid cartridge assembly, or similar, requiring replacement; (v) receiving and/or providing to a user, alerts and notifications related to preset times for usage of the vaporizer; and, (w) receiving and/or providing to a user, heating element alerts and notifications status or "health"-such as number of cycles performed, and/or number of cycles remaining before suggested and/or required replacement of a heating element or heating element assembly.

The power settings of the vaporizer may be set and/or specified to modulate or configure the activation energy delivered to the heating element(s) as well as modulating or configuring the parameters of the heating element(s) being energized in relation to the time to peak activation or "warm up" or "ramp", and/or the time of maximum or peak activation, and/or the time of the heating element being deactivated or the "cool down" to effect and modulate vapor and/or aerosol strength, vapor and/or aerosol density, vapor and/or aerosol volume, vapor and/or aerosol flavor, vapor and/or aerosol temperature, and/or similar vapor and aerosol characteristics of the vapor or aerosol generated by the vaporizer. In an implementation, the power settings of the vaporizer may be set and/or specified such that the user can make setting adjustments to the vaporizer to maximize battery life. In this case, the vaporizer may resultantly operate at lower energy output to preserve the maximum number of cycles that can be sustained per battery charge cycle. Conversely the power settings of the vaporizer may be set and/or specified such that the user can maximize performance in relation to the energy output of the device per cycle.

Cartridge-related settings of the vaporizer can be based on information about the cartridge, including liquid components and/or formulation, or similar such that the information relating to the liquid may be vaporized or aerosolized. The liquid related settings of the vaporizer can have predetermined as well as user configurable settings to modulate, configure, adjust or otherwise configure the device activation parameters. In an implementation, settings related to user specific environmental configurations can be made such that the vaporizer optimizes heating element activation and activation parameters to optimize performance based on ambient temperature, humidity, and/or altitude. For example, the vaporizer may have configurations such as cold weather or warm weather settings, humidity settings, and/or altitude settings.

A vaporizer may be configured (programmed) with time based settings, such as for example, user specific temporal configurations such as the user preferring higher active component delivery per inhalation at specific times of the day. A vaporizer can be configured such that the vaporizer delivers dosages of an active component based on the time of day. For example, the vaporizer can be configured such that the dosage delivered to the user is highest, or at maximum value (or similar) in the evening and is held at a lower delivered dose per inhalation, or minimum value (or similar) earlier in the day. The user can program these settings (and others described herein) based on personal preference.

Taste and/or flavor related settings of the vaporizer can minimize, maximize, and or modulate functional effects of the taste and/or flavor component of the vapor product. For example, the vaporizer can be configured to activate in such a way that the flavor delivered from the vapor or aerosol is minimized, maximized, or modulated over the period of an inhalation. Some components of the liquid being vaporized that may contribute to the flavor characteristics of the vapor or aerosol may be more profound, more prevalent, or more substantial when the vaporizer is activated with higher temperature ranges being generated by the heating element than when lower temperature ranges are being generated by the heating element (within the range of temperatures that the heating element may operate in order to generate a vapor or aerosol for inhalation by the user). For example, the user may set the vaporizer to perform for maximal, minimal, moderate, or another interim value of flavor for the vapor or aerosol product. The vaporizer may modulate the heating element activation cycle accordingly.

Functional effect-related setting of the vaporizer can minimize, maximize, or modulate the functional effects related to pharmacodynamics and pharmacokinetics of an active ingredient or drug component of the vapor or aerosol product. For example, the vaporizer can be configured to activate in such a way that the active component or drug delivered from the vapor or aerosol is minimized or maximized in terms of target tissue or organ delivery. Particle size may be modulated. A user may be using a vaporizer for the delivery of nicotine as the active or drug component in the vapor or aerosol. It may be desirable for (or by) the user to have an option for more rapid delivery of the nicotine to the bloodstream-such as after a period of not having nicotine (when the user's urge or craving is likely to be elevated). Alternatively, at times it may be desirable for (or by) the user to have a slower absorption of nicotine into the blood stream such as at times when: (i) the user's craving or urge is low, (ii) when the user wants to have a more prolonged period of time before they have the urge or craving for nicotine-such as prior to going to sleep, or an event where they will be unable to use the device for dosing or administration of the nicotine. The vaporizer settings relating to the activation of the device and the temperature of the heating element and heating element activation characteristics may be modulated such that, for example, at lower temperature activation the particle size of the drug component is larger than at times of a higher temperature activation of the heating element. Thus, by modulating the input of thermal or heat energy inputted into the vaporization chamber by the heating element to volatize or vaporize the liquid containing the active component(s) or drug(s), the characteristics of the vapor or aerosol in relation to the particle size of the active component(s) or drug(s) can be wholly or partially modulated by the user. These settings can also be used by the end user or healthcare provider (or similar) to reduce dependence on the active component(s) or drug(s) such as nicotine. This transition can also be used in conjunction with nicotine dosage reduction for reducing or mitigating the user's nicotine dependence or addiction.

An app may receive alerts and notifications associated with the vaporizer. These alerts and notifications can include, for example: battery life status, battery condition data (such as number of battery cycles), and battery "health" (such that the user can be notified, as desired, to the current and "real time" overall condition of the vaporizer internal battery(ies)).

A vaporizer and/or an associated application (app) running on a digital consumer technology product (e.g., a device that forms or is part of a vaporizer system as described above) may share data with a manufacturer, manufacturer affiliate, or other entity (retailer, healthcare provider, supplier, marketing entity, etc.). A vaporizer and/or an associated application may gather, receive, log, store, transmit, extrapolate, and/or the like, anonymous or user specific usage data-such as frequency of use. A vaporizer and/or an associated application can gather, receive, log, store, transmit, extrapolate, and/or the like, user specific usage data such as activation cycle characteristics, such as duration of activations and user specified activation settings (if applicable.) A vaporizer and/or an associated application can gather, receive, log, store, transmit, extrapolate, and/or the like, user specific demographic information. A vaporizer and/or an associated application can gather, receive, log, store, transmit, extrapolate, and/or the like, user specific socioeconomic information. A vaporizer and/or an associated application can gather, receive, log, store, transmit, extrapolate, and/or the like, user specific information. A vaporizer and/or an associated application can gather, receive, log, store, transmit, extrapolate, and/or the like, user specific feedback information. A vaporizer and/or an associated application can gather, receive, log, store, transmit, extrapolate, and/or the like, user specific demographic information. A vaporizer and/or an associated application can gather, receive, log, store, transmit, extrapolate, and/or the like, user specific feedback information using surveys, polls, and the like, and/or data analytics.

A vaporizer and/or an associated application can gather, receive, log, store, transmit, extrapolate, and/or the like, anonymous and/or user specific usage and/or reliability data such as device errors or malfunctions. A vaporizer and/or an associated application can gather, receive, log, store, transmit, extrapolate, and/or the like, user specific usage and/or reliability data such as requests for warranty services, repairs, and or replacements, etc. A vaporizer and/or an associated application can gather, receive, log, store, transmit, extrapolate, and/or the like, user specific customer satisfaction data such as requests for technical support. A vaporizer and/or an associated application can gather, receive, log, store, transmit, extrapolate, and/or the like, user specific sales lead data such as requests for product information. A vaporizer and/or an associated application can gather, receive, log, store, transmit, extrapolate, and/or the like, user specific usability data such as requests for usage instructions. A vaporizer and/or an associated application can gather, receive, log, store, transmit, extrapolate, and/or the like, user specific information such as requests for information on product features or functions. A vaporizer and/or an associated application can gather, receive, log, store, transmit, extrapolate, and/or the like, user specific marketing data such as requests for information on purchasing a vaporizer and/or acquiring a vaporizer by way of a prescription from a physician or healthcare provider.

A vaporizer and/or an associated application can gather, receive, log, store, transmit, extrapolate, and/or the like, vaporizer data indicating misuse or abuse of the vaporizer. A vaporizer and/or an associated application can gather, receive, log, store, transmit, extrapolate, and/or the like, vaporizer and/or use data and/or data transmission features that can be used to locate the vaporizer. The vaporizer and/or an associated application can gather, receive, log, store, transmit, extrapolate, and/or the like, data and/or data transmission features that can be used to locate the vaporizer if it is lost or stolen. A vaporizer, via an associated application, can gather, receive, log, store, transmit, extrapolate, and/or the like, notifications regarding product recalls or similar issues and/or inform the user of such recalls or issues. A vaporizer, via an associated application, can gather, receive, log, store, transmit, extrapolate, data sharing, and/or the like, notifications regarding manufacturer terms and conditions (e.g., cartridge manufacturer) and/or inform the user of such terms and conditions, and/or receive approval of such terms and conditions from the user.

A vaporizer, via an associated application running on a device that is part of a vaporizer system, can gather, receive, log, store, transmit, extrapolate, data share, and/or the like, data from a network that may be used to identify, contact, or connect with other users of vaporizers, and may, via an associated application, gather, receive, log, store, transmit, extrapolate, data share, and/or the like, data from a network that may be used to identify, contact, or connect with other users within the network. The vaporizer may select and/or authorize the sharing of all or some of the data gathered, received, logged, stored, transmitted, extrapolated, shared, or the like by the vaporizer, or gathered directly from the user using applications associated with the vaporizer. A vaporizer may select and/or authorize the sharing, via a network, of all or some of the data gathered, received, logged, stored, transmitted, extrapolated, shared, or the like by the vaporizer, or gathered directly from the user using applications associated with the vaporizer. The network may comprise social media. The social media membership may comprise a user's family. The social media membership may comprise a user's friends. The social media membership may comprise a support group or similar (e.g., quit smoking group). The social media membership may comprise a third-party service, company, organization (e.g., church), other users of the vaporizer, or the like.

A vaporizer, and/or an associated application can gather, receive, log, store, transmit, extrapolate, and/or the like, data useful to perform software configuration of the device and or the device application(s). A vaporizer and/or an associated application can gather, receive, log, store, transmit, extrapolate, and/or the like, data useful or required to perform software configuration of the vaporizer and/or the associated application(s). A vaporizer and/or an associated application can gather, receive, log, store, transmit, extrapolate, and/or the like, data useful or required to perform software configuration of the vaporizer, and/or the associated application(s) where the software is configured by the manufacturer or manufacturer's subsidiary or representatives or third party or similar. A vaporizer and/or an associated application can gather, receive, log, store, transmit, extrapolate, and/or the like, data useful or required to perform third party software configuration of a vaporizer and/or the associated application(s). A vaporizer and/or an associated application can gather, receive, log, store, transmit, extrapolate, and/or the like, data useful or required to perform firmware updates of the vaporizer, and/or the associated application(s). A vaporizer and/or an associated application can provide for the notification of the user via a vaporizer, and/or an associated application that a firmware or similar updates to the vaporizer and/or an associated application is available and/or required for trouble shooting the device or remediating a problem or issue with the vaporizer, and/or an associated application which is preventing some aspect of intended or proper function(s) of the vaporizer and/or an associated application. A vaporizer and/or an associated application can provide for the notification of the user via the vaporizer and/or an associated application that a firmware or similar update to the vaporizer and/or an associated application is available and/or required for providing additional functions relating to or intended to improved vaporizer performance, enhance user experience, or similarly improve some aspect of intended or proper function(s) of the vaporizer and/or an associated application.

A vaporizer and/or an associated application can share data gathered by the vaporizer, or gathered directly from the user using the application with the user's healthcare provider. A vaporizer and/or an associated application can share data gathered by the vaporizer, or gathered directly from the user using the application with the user's healthcare network. A vaporizer and/or an associated application can share data gathered by the vaporizer or gathered directly from the user using the application with the user's insurance provider. A vaporizer and/or an associated application can share data gathered by the vaporizer, or gathered directly from the user using the application with the user's pharmacy and/or prescription drug provider, or the like. A vaporizer and/or an associated application can depersonalize or otherwise make anonymous data gathered by the vaporizer or gathered directly from the user so that the depersonalized data can be shared or used for purposes such as research, analysis, publication, or similar purposes.

A vaporizer and/or an associated application can provide for the notification of the user via the vaporizer and/or the associated application of the availability of a prescription issued or written for the end user being ready for pick-up, delivery, shipment to the user or similar of a prescription component intended for delivery to the patient by a vaporizer. For example, a pharmacy may send a notification to the user, via the vaporizer and/or an associated application, such as to notify the user that their prescription for a vaporizer or vaporizable material (e.g., cartridges or liquids) is available for the user to pick up from the pharmacy (other commercial venues, not limited to pharmacies, may also do this, including shops, dispensaries, etc.). A vaporizer and/or an associated application can allow for healthcare providers, networks, agents, authorized third parties or similar entities to send alerts, messages, surveys, or similar to the user via the vaporizer and/or the associated application. A vaporizer and/or an associated application can allow for healthcare providers, networks, agents, authorized third parties or similar entities to access data that is generated as a result of surveys, or similar through the vaporizer and/or the associated application.

A vaporizer and/or an associated application can authorize (e.g., allow) a healthcare provider to configure, adjust, modulate, and/or manipulate vaporizer settings. A vaporizer and/or an associated application can authorize a healthcare provider to configure, adjust, modulate, and/or manipulate vaporizer settings which the user is not authorized to change, alter, reconfigure or change the settings, configurations, etc. made by the healthcare provider. A vaporizer and/or an associated application can authorize a representative or agent of the healthcare provider to configure, adjust, modulate, and/or manipulate vaporizer settings which the user is not authorized to change, alter, reconfigure or change the settings, configurations, etc. made by the representative or agent of the healthcare provider.

A vaporizer and/or an associated application can share user specific information, such as end user ownership of products relating to the device, device components, device accessories or similar data, gathered by the vaporizer or gathered directly from the user through the use of the application. A vaporizer and/or an associated application can share user specific information, such as end user purchasing of products relating to the device, device components, device accessories or similar data, gathered by the vaporizer or gathered directly from the user through the use of the application. A vaporizer and/or an associated application can provide for the notification of the user via the vaporizer and/or the associated application of notifications from retailer(s) or similar regarding product promotions. A vaporizer and/or an associated application can provide for the notification of the user via the vaporizer and/or the associated application similar of notifications from retailer(s) or similar regarding product availability. A vaporizer and/or an associated application can provide for the notification of the user via the vaporizer and/or the associated application similar of notifications from retailer(s) or similar regarding release of new product or accessories.

A vaporizer and/or an associated application can use demographic or similar location services to find retail locations in geographic proximity of the user. A vaporizer and/or an associated application can gather, receive, log, store, transmit, extrapolate, and/or the like, data relating to device purchasing, device accessories purchasing, vaporizer liquid and associated packaging or assembly purchasing, frequency of purchasing, point of sale, discounts applied by user when purchasing, and related or similar information. A vaporizer and/or an associated application can gather, receive, log, store, transmit, extrapolate, and/or the like, data relating to device purchasing, device accessories purchasing, vaporizer liquid and associated packaging or assembly purchasing, frequency of purchasing, point of sale, discounts applied, and related or similar information.

A vaporizer and/or an associated application can provide incentives to the user to share information relating to device purchasing, device accessories purchasing, vaporizer liquid and associated packaging or assembly purchasing, frequency of purchasing, point of sale, discounts applied and related information such as discounts, coupons, promotional codes, free items, or similar. A vaporizer and/or an associated application can provide for the use of the user profile to provide targeted incentives to the user to share information relating to device purchasing, device accessories purchasing, vaporizer liquid and associated packaging or assembly purchasing, frequency of purchasing, point of sale, discounts applied, promotional codes used, and related information such as discounts, coupons, free items, or similar.

A vaporizer and/or an associated application can render the vaporizer inactive and unable to be used, as mentioned above. For example, a vaporizer and/or an associated application can render the vaporizer inactive and unable to be used if a malfunction or similar has occurred. A vaporizer and/or an associated application can render the vaporizer inactive and unable to be used until the authorized user enters a Personal Identification Number (PIN) using the application which then activates the vaporizer. A vaporizer and/or an associated application can render the vaporizer inactive and unable to be used until the authorized user has a biometric identifier that when recognized or confirmed or verified or similar, using the application, activates the vaporizer. As discussed above, unauthorized use of a vaporizer and/or an associated application can be prevented by using PIN and/or unique biometric identifier. A vaporizer and/or an associated application can save device data and personal settings for individual users so that more than one user may use the vaporizer. A vaporizer and/or an associated application can save device data and personal settings to be saved for individual users where the settings for device data and personal settings for different users can be applied to the vaporizer and the intended user through the application. The user may select their saved configurations for a vaporizer and the respective device will operate under that user selected configuration. A vaporizer and/or an associated application can have the ability for the user or users to have one or more of user settings and/or configurations that are saved and can be selected by users. A vaporizer and/or an associated application can have the ability to allow saved user settings and personal settings or configurations to be shared by the user through the application and/or an associated network. A vaporizer and/or an associated application can allow other user settings and/or configurations to be shared with the user through the application or an associated network.

A vaporizer and/or an associated application can facilitate, prompt, or the like, a user to rate (such as through common methods such a 1-10 where "10" is the best, or 1-5 "stars" where "5" stars is the best) their vaporizer, vaporizer configurations, cartridge (e.g., particular flavor or brand of cartridges, etc.), or the like. A vaporizer and/or an associated application can facilitate, prompt, or the like, the user to rate other user configurations. A vaporizer and/or an associated application can share and access a database of user configurations that may or may not have ratings and be able to access the user configurations through the application and download user configurations for use in the user's own device. A vaporizer and/or an associated application can have the ability to share and access a database of user configurations that may or may not have ratings and be able to access the user configurations through the application and upload their user configurations for use in other users' devices.

A vaporizer and/or an associated application can share user data with the manufacturer, manufacturers subsidiaries, manufactures agents, or a third party for generating user profiles based on user specific usage data, demographic data, socioeconomic data or similar. A vaporizer and/or an associated application can have the ability to utilize user data shared with the manufacturer, manufacturer's subsidiaries, manufacturer's agents, or a third party to determine specific user profiles.

A vaporizer and/or an associated application can allow, facilitate, authorize, confirm or similar the sharing of data between the associated application and other application(s) that may be installed or a component of the user's personal digital device. A vaporizer and/or an associated application can share information and/or data with a social media application. A vaporizer and/or an associated application can share information and/or data with email service, email provider, email hosting, or similar applications. A vaporizer and/or an associated application can share information and/or data with text message, short message service (SMS), or similar applications. A vaporizer and/or an associated application can share information and/or data with a location based services application. A vaporizer and/or an associated application can share information and/or data with a map or mapping, navigation, location or similar application. A vaporizer and/or an associated application can share information and/or data with healthcare, healthcare provider, healthcare services, healthcare network or similar application. A vaporizer and/or an associated application can share information and/or data with pharmacy, pharmacy type service provider or similar application. A vaporizer and/or an associated application can share information and/or data with a weather, weather forecasting, weather reporting or similar application. A vaporizer and/or an associated application can share information and/or data with the device manufacturer's application. A vaporizer and/or an associated application can share information and/or data with a research or a research orientated application. A vaporizer and/or an associated application can share information and/or data with a vaporizer retailer or similar consumer device application.

A vaporizer and/or an associated application can have the ability to authorize or allow data gathering, receiving, logging, storing, transmission, extrapolation or similar for the purpose of the device or associated application sending error codes or error reports to the manufacturer, manufacturer's subsidiaries, manufacturer's agents, or a third party for the purpose of addressing problems with device performance or function. A vaporizer and/or an associated application can have the ability to authorize or allow data gathering, receiving, logging, storing, transmission, extrapolation or similar for the device or associated application to send error codes or error reports to the manufacturer, manufacturer's subsidiaries, manufacturer's agents, or a third party for the purpose of addressing problems with device application(s). A vaporizer and/or an associated application can have the ability to authorize or allow data gathering, receiving, logging, storing, transmission, extrapolation or similar for the device or device application to send error codes or error reports to the manufacturer, manufacturer's subsidiaries, manufacturer's agents, or a third party for the purpose of extrapolating data metrics that relate to device malfunctioning. A vaporizer and/or an associated application can have the ability to authorize or allow data gathering, receiving, logging, storing, transmission, extrapolation or similar for the purpose of the device or associated application sending error codes or error reports to the manufacturer, manufacturer's subsidiaries, manufacturer's agents, or a third party for the purpose of gathering, receiving, logging, storing, transmission, extrapolation or similar of data that may relate to manufacturing, quality control or similar issues or potential problems related to the device, device components, or liquid being used in the device. A vaporizer and/or an associated application can have the ability to gather, receive, log, store, transmit, extrapolate, or similar, data for troubleshooting device issues or problems. A vaporizer and/or an associated application can have the ability to gather, receive, log, store, transmit, extrapolate, or similar, data for troubleshooting device issues or problems that may relate to user error.

A vaporizer and/or an associated application can have the ability to use methods of data transmission such as wireless and wired technologies. A vaporizer and/or an associated application can have the ability to use methods of data transmission such as wireless and wired technologies to perform one or more of the functions, capabilities, methods, abilities, etc., described herein. A vaporizer and/or an associated application can have the ability to use methods of data transmission such as WiFi, Bluetooth, cellular, 3G, 4G, near field communication (NFC), or similar for the transmission of data to the user's personal digital device. Such communications, may occur through establishment of a wireless communication channel between first communication hardware of a device and second communication hardware of a vaporizer. A vaporizer and/or an associated application can have the ability to use methods of data transmission such as Wi-Fi, Bluetooth, cellular, 3G, 4G, near field communication (NFC), or similar for the transmission of data to a network. Accordingly, the first communication hardware and the second communication hardware can include circuitry and one or more transceivers configured for at least one of these (or other comparable) communication approaches. A vaporizer and/or an associated application can have the ability to use methods of data transmission such as text messaging or SMS. A vaporizer and/or an associated application can have the ability to use methods of data transmission such as electronic mail or email. A vaporizer and/or an associated application can have the ability to use methods of data transmission such as notifications or push notifications to the user's digital device, which can include the first communication hardware.

A vaporizer and/or an associated application can include features (e.g., software-based buttons or controls and/or physical input devices or controls) that enable user control of the functionality, features, configurations etc. of a vaporizer and/or an associated application using various features of the application referred to as configurations or settings. These settings can include, but are not limited to exemplary general usage settings such as: (a) desired number of activations cycles over a period of time; (b) configuring and or setting reminders, alarms, or similar to notify the user; (c) desired dose delivery of active substance per inhalation; (d) desired total delivered dose over a period of time, such as a total daily dose; (e) power settings of vaporizer to modulate the vapor or aerosol strength, vapor or aerosol density, vapor or aerosol volume, vapor or aerosol flavor, vapor or aerosol temperature or similar vapor or aerosol characteristics of the vapor or aerosol generated by the device (the power settings could modulate or configure the activation energy delivered to the heating element(s) as well as modulate or configure the parameters of the heating element(s) being energized in relation to the time to peak activation or "warm up" or "ramp", and or the time of maximum or peak activation, and or the time of the heating element being deactivated or the "cool down" to effect and modulate the vapor or aerosol strength, vapor or aerosol density, vapor or aerosol volume, vapor or aerosol flavor, vapor or aerosol temperature or similar characteristics of the vapor or aerosol generated by the device); (f) power settings of vaporizer to modulate, adjust, configure or similar the settings of the device as they relate to battery life and performance such that the user can make setting adjustment to the device to maximize battery life and the device will resultantly operate at lower energy output to preserve the maximum number of cycles that be sustained per battery charge cycle (conversely the user could modulate, adjust, configure or similar the settings of the device to maximize performance in relation to the energy output of the device per cycle); (g) settings related to the liquid components and formulation or similar such that the information relating to the liquid to be vaporized or aerosolized can have predetermined as well as user configurable settings to modulate, configure, adjust or similar vaporizer activation parameters; (h) settings related to user specific environmental configurations such as cold weather or warm weather settings such that the device optimizes heating element activation and activation parameters to optimize performance based on ambient temperature; (i) settings related to user specific environmental configurations such as high or low humidity settings such that vaporizer optimizes heating element activation and activation parameters to optimize performance based on user locale humidity values or ranges; (j) settings related to user specific environmental configurations such as user locale altitude settings such that vaporizer optimizes heating element activation and activation parameters to optimize performance based on end user altitude; (k) settings related to user specific temporal configurations such as the user preferring higher active component delivery per inhalation at specific times of the day (for example, vaporizer can be configured such that it delivers higher dosage of active component related to a time of day such that the dosage delivered to the user is highest, or at maximum value or similar, in the morning and tapers down to a lower delivered dose per inhalation, or minimum value, or similar at the end of the evening); (I) settings related to modulating vaporizer performance and activation parameters to minimize or maximize the functional effects of the taste or flavor component of the vapor product such that the vaporizer can be configured to activate in such a way that the flavor delivered from the vapor or aerosol is minimized or maximized (for example components of the liquid being vaporized that may contribute to the flavor characteristics of the vapor or aerosol may be more profound, or more prevalent, or more substantial when vaporizer is activated with higher temperature ranges being generated by the heating element than when lower temperature ranges are being generated by the heating element within the range of temperatures that the heating element may operate within in order to generate a vapor or aerosol for inhalation by the user); for example the user may set vaporizer to perform for maximal, minimal, moderate, or another interim value of flavor for the vapor or aerosol product and the heating element activation cycle will be modulated accordingly; (m) settings related to modulating vaporizer performance and activation parameters to minimize or maximize the functional effects related to pharmacodynamics and pharmacokinetics of the active or drug component of the vapor or aerosol product such that vaporizer can be configured to activate in such a way that the active component or drug delivered from the vapor or aerosol is minimized or maximized in terms of target tissue or organ delivery; (n) device alerts and notifications such as battery life status and battery condition(s) data such as number of battery cycles and battery "health" such that the user can be notified as desired to the current in real time and overall condition of the device's internal battery, and the device's charging case internal battery; (o) device alerts and notifications such as the vaporizer battery requiring recharging; (p) device alerts and notifications such as vaporizer battery being fully charged; (q) device alerts and notifications such as liquid cartridge status, such as number of usages or inhalations taken and number or usages remaining; (r) device alerts and notifications such as liquid cartridge contents such as active component(s) and strength or dosage or similar, and flavor profile or similar, and general formulation; (s) device alerts and notifications such as liquid cartridge or liquid cartridge assembly or similar requiring replacement; (t) device alerts and notifications such as predetermined or preset times for usage of vaporizer; (u) device alerts and notifications such as device heating element status or "health" such as number of cycles performed and number of cycles remaining before suggested or required replacement of heating element or heating element assembly.

Settings can include, but are not limited to device manufacturer data sharing settings such as: (a) Anonymous or user specific usage data such as frequency of use; (b) Anonymous or user specific usage data such as activation cycle characteristics such as duration of activations and user specified activation settings if applicable; (c) User specific data such as demographic information; (d) User specific data such as socioeconomic information; (e) User specific data such as user feedback through the use of surveys or similar; (f) Anonymous or user specific usage data such device errors or malfunctions; (g) User specific data such as requests for warranty services or repairs or replacements or similar; (h) User specific data such as requests for technical support; (i) User specific data such as requests for product information; (j) User specific data such as requests for usage instructions; (k) User specific data such as requests for information on product features or functions; (l) User specific data such as requests for information on purchasing product or acquiring the product through a prescription from a physician or healthcare provider; (m) Device data indicating misuse or abuse of the device; (n) Device data and data transmission features used to locate the device if the device is lost or stolen; (o) Notifications to the user through the device or application(s) relating to product recall(s) or similar issues; (p) General data sharing to manufacture terms and conditions recognition and user agreement to said terms.

Settings can include, but are not limited to user, usage, system, device, and operational data settings such as: (a) Settings relating to selecting and authorizing the sharing of all or some of the data gathered by the device or gathered directly from the user through the use of an application(s) to a network(s); (b) Where network(s) may be social media; (c) Where network(s) may be comprised of the user's family and or friends; (d) Where network(s) may be comprised of a support group or similar; (e) Settings relating to the use of the sharing of data over a network(s) that may be used to identify, contact, or connect with other users of the device; (f) Where other network(s) may be a third party service, company, organization or similar.

Settings can include, but are not limited to software configuration and firmware updating settings such as: (a) Settings relating to the sharing and transmission of data required or useful to perform software configuration of the device and or the device application(s); (b) Settings relating to the sharing and transmission of data required to perform software configuration of the device and or the device application(s) where the software is configured by the manufacturer or manufacturers subsidiary or representatives or third party or similar; (c) Settings relating to the sharing and transmission of data required to perform software configuration of the device and or the device application(s) where the software is configured by a third party; (d) Settings relating to the authorization for the sharing and transmission of data required to perform firmware or similar updates to the device and or application; (e) Settings relating to the notification of the user through the device or application(s) that a firmware or similar updates to the device and or application(s) is available and or required; (f) Settings relating to the notification of the user through the device or application(s) that a firmware or similar updates to the device and or application(s) is available and or required as a means of trouble shooting the device or remediating a problem or issue with the device or application(s) preventing some aspect of intended or proper function(s).

Settings can include, but are not limited to healthcare system data sharing settings such as: (a) Settings relating to the sharing of all or some of the data gathered by the device or gathered directly from the user through the use of application(s) to the user's healthcare provider; (b) Settings relating to the sharing of all or some of the data gathered by the device or gathered directly from the user through the use of application(s) to the user's healthcare network; (c) Settings relating to the sharing of all or some of the data gathered by the device or gathered directly from the user through the use of application(s) to the user's insurance provider; (d) Settings relating to the sharing of all or some of the data gathered by the device or gathered directly from the user through the use of application(s) to the user's pharmacy or prescription drug provider or similar; (e) Settings relating to the notification of the availability of a prescription issued or written for the end user being ready for pick-up, delivery, and/or shipment to the user or similar of a prescription component intended for delivery to the patient by the device. For example, a pharmacy could send a notification to the user, through the device application, such as to notify the user that their prescription for the device or device components is available for the user to pick up from the pharmacy; (f) Settings relating to the authorization of a healthcare provider to configure, adjust, modulate, manipulate or similar the device settings; (g) Settings relating to the authorization of a healthcare provider to configure, adjust, modulate, manipulate or similar the device settings where the user is not authorized to change, alter, reconfigure or similar the settings, configurations, or similar made by the healthcare provider; (h) Settings authorizing a representative or agent or similar of the healthcare provider to configure, adjust, modulate, manipulate or similar the device settings where the user is not authorized to change, alter, reconfigure or similar the settings, configurations, or similar made by the healthcare representative or agent or similar; (i) Settings allowing for data shared with the healthcare provider or network to be depersonalized or otherwise made anonymous and used for other purposes such as research, analysis, publication, or similar purposes; (j) Settings allowing for healthcare providers, networks, agents, authorized third parties or similar to send alerts, messages, surveys, or similar through the device application(s); (k) Settings allowing for healthcare providers, networks, agents, authorized third parties or similar to access data that is generated as a result of surveys, or similar through the device application(s).

Settings can include, but are not limited to retailer and/or consumer facing data settings such as: (a) Settings relating to the sharing user specific information such as product, device, component, accessories or similar details; (b) Settings relating to receiving notifications from retailer(s) or similar regarding product promotions; (c) Settings relating to receiving notifications from retailer(s) or similar regarding product availability; (d) Settings relating to receiving notifications from retailer(s) or similar regarding release of new product or accessories; (e) Settings relating to using demographic or similar location services to find retail locations in geographic proximity of the user; (f) Settings relating to the sharing of data that may be used for demographic, socioeconomic, or similar marketing or promotional activities; (g) Settings relating to the gathering of data relating to device purchasing, device accessories purchasing, vaporizer liquid and associated packaging or assembly purchasing, frequency of purchasing, point of sale, discounts applied by user when purchasing, and related or similar information; (h) Settings relating to the sharing of data relating to device purchasing, device accessories purchasing, vaporizer liquid and associated packaging or assembly purchasing, frequency of purchasing, point of sale, discounts applied, and related or similar information; (i) The use of the application to provide incentives to the user to share information relating to device purchasing, device accessories purchasing, vaporizer liquid and associated packaging or assembly purchasing, frequency of purchasing, point of sale, discounts applied and related information such as discounts, coupons, promotional codes, free items, or similar; (j) Settings relating to the use of the user profile to provide targeted incentives to the user to share information relating to device purchasing, device accessories purchasing, vaporizer liquid and associated packaging or assembly purchasing, frequency of purchasing, point of sale, discounts applied, promotional codes used, and related information such as discounts, coupons, free items, or similar.

Settings can include, but are not limited to device access settings such as: (a) Settings relating to rendering the device inactive and unable to be used; (b) Settings relating to rendering the device inactive and unable to be used where the authorized user has a Personal Identification Number (PIN) that when entered using the application activates the device; (c) Settings relating to rendering the device inactive and unable to be used where the authorized user has a biometric identifier that when recognized or confirmed or verified or similar using the application activates the device; (d) Settings relating to rendering the device inactive and unable to be used where the authorized user has a biometric identifier that when recognized or confirmed or verified using the application activates the device where the biometric identifier is a fingerprint; (e) Settings relating to rendering the device inactive and unable to be used where the authorized user has a biometric identifier that when recognized or confirmed or verified using the application activates the device where the biometric identifier is an eye or iris or similar scan; (f) Settings relating to rendering the device inactive and unable to be used where the authorized user has a biometric identifier that when recognized or confirmed or verified using the application activates the device where the biometric identifier is facial recognition; (g) Settings where unauthorized use of the device is prevented by using PIN or unique biometric identifier; (h) Settings relating to the sharing of data relating to the attempted unauthorized use of the device; (i) Settings relating to the sharing of data over a network to authorize the user and activate the device; (j) Settings relating to sharing of data such that biometric authentication can be performed through the use of a network; (k) Settings related to the time or duration of time that passes after use before the device is rendered inactive and authentication is required to authorize the device; (I) Settings related to the resetting or changing of user specific authentication information such as the PIN.

Settings can include, but are not limited to multiple user settings such as: (a) Settings relating to the sharing and transmission of data required or useful to perform software configuration of the device and or the device application(s); (b) Settings relating to the sharing and transmission of data required to perform software configuration of the device and or the device application(s) where the software is configured by the manufacturer or manufacturer's subsidiary or representatives or third party or similar; (c) Settings relating to the sharing and transmission of data required to perform software configuration of the device and or the device application(s) where the software is configured by a third party; (d) Settings relating to the authorization for the sharing and transmission of data required to perform firmware or similar updates to the device and or application; (e) Settings relating to the notification of the user through the device or application(s) that a firmware or similar updates to the device and or application(s) is available and/or required; (f) Settings relating to the notification of the user through the device or application(s) that a firmware or similar updates to the device and or application(s) is available and or required as a means of trouble shooting the device or remediating a problem or issue with the device or application(s) preventing some aspect of intended or proper function(s).

Settings can include, but are not limited to defined usage profile settings such as: (a) Settings related to the sharing of user data to the manufacturer, manufacturer's subsidiaries, manufacturer's agents, or a third party for the purpose of generating user profiles based on user specific usage data, demographic data, socioeconomic data or similar; (b) Where the use of user data shared with or sent to the manufacturer, manufacturer's subsidiaries, manufacturer's agents, or a third party for the purpose of generating user profiles based on user specific usage data, demographic data, socioeconomic data or similar is utilized to determine specific user profiles; (c) Where the user profiles are a group of setting configurations that correlate to a specific subset of users; (d) Where a subset of users may be based on demographic data, socioeconomic, personal data gathered through the use of the application, device usage data or similar; (e) Where user profiles may be specific to the subset of users and recommended device configuration based on user profile data could be available to the user of the device based on the user's similarities to a subset of users; (f) Where the user experience is optimized by using cumulative data from similar users to establish a default setting configuration for the device based on the user's demographic data, socioeconomic data or similar.

Settings can include, but are not limited to settings related to integration with other applications such as: (a) Settings to allow, facilitate, authorize, confirm or similar the sharing of data between the device application and other application(s) that may be installed or a component of the user's personal digital device; (b) Where other application(s) that the device application shares information with may be social media application(s); (c) Where other application(s) that the device application shares information with may be email service, email provider, email hosting, or similar application(s); (d) Where other application(s) that the device application shares information with may be text message, SMS, or similar application(s); (e) Where other application(s) that the device application shares information with may be location services application(s); (f) Where other application(s) that the device application shares information with may be map or mapping, navigation, location or similar application(s); (g) Where other application(s) that the device application shares information with may be healthcare, healthcare provider, healthcare services, healthcare network or similar application(s); (h) Where other application(s) that the device application shares information with may be pharmacy, or pharmacy type service provider or similar application(s); (i) Where other application(s) that the device application shares information with may be weather, or weather forecasting, or weather reporting or similar application(s); (j) Where other application(s) that the device application shares information with may be the device manufacturers application(s); (k) Where other application(s) that the device application shares information with may be research or research orientated application(s); (l) Where other application(s) that the device application shares information with may be device retailer or similar consumer device application(s).

Settings can include, but are not limited to error code and troubleshooting such as: (a) Settings relating to the authorization or allowance of data sharing for the purpose of the device or device application sending error codes or error reports to the manufacturer, manufacturer's subsidiaries, manufacturer's agents, or a third party for the purpose of addressing problems with device performance or function; (b) Settings relating to the authorization or allowance of data sharing for the purpose of the device or device application sending error codes or error reports to the manufacturer, manufacturer's subsidiaries, manufacturer's agents, or a third party for the purpose of addressing problems with device application(s); (c) Settings relating to the authorization or allowance of data sharing for the purpose of the device or device application sending error codes or error reports to the manufacturer, manufacturer's subsidiaries, manufacturer's agents, or a third party for the purpose of extrapolating data metrics that relate to device malfunctioning; (d) Settings relating to the authorization or allowance of data sharing for the purpose of the device or device application sending error codes or error reports to the manufacturer, manufacturer's subsidiaries, manufacturer's agents, or a third party for the purpose of gathering data that may relate to manufacturing, or quality control or similar issues or potential problems related to the device, device components, or liquid being used in the device; (e) Settings relating to the sharing of data for the purpose of troubleshooting device issues or problems. (f) Settings relating to the sharing of data for the purpose of troubleshooting device issues or problems that may relate to user error.

Settings can include, but are not limited to settings related to methods of communication such as: (a) Settings relating to the device or device application using methods of data transmission such as wireless and wired technologies; (b) Settings relating to the device or device application using methods of data transmission such as WiFi, Bluetooth, or similar for the transmission of data to the user's personal digital device; (c) Settings relating to the device or device application using methods of data transmission such as wired or wireless methods or similar for the transmission of data to a network; (d) Settings relating to the device or device application using methods of data transmission such as text messaging or SMS; (e) Settings relating to the device or device application using methods of data transmission such as electronic mail or email; (f) Settings relating to the device or device application using methods of data transmission such as notifications or push notifications on the user's digital device.

The application can be used to provide information on trouble shooting the device in the event of a performance issue or malfunction. The application can be used to provide safety information relating to the device or to the user. The application can be used to provide safety information relating to the maintenance, cleaning, or similar activities for the device. The application can be used to provide storage information for the device. The application can be used to provide information relating to the disposal or recycling of the device. The application can be used to provide information on the proper disassembly and assembly of the device. The application can be used to provide information such as the manufacturers, distributors, retailers, or similar website and or contact information. The application can be used to provide information such as a website uniform resource locator (URL) or link for internet forums that may relate to the use, troubleshooting, user experience, user reviews or similar. The application can be used to provide safety information relating to the device to the user. The application can be used to provide information on available products, accessories, or similar that may be related to the device. The application can be used to provide a space for advertising consumer products or services that may be related to the device. The application can be used to provide functions relating to personal user goals for device usage and to track usage as it relates to the users defined goals and to present the data in the forms of charts, graphs, or similar.

The systems, controller, and functions described above may be implemented with or executed by one or more computer systems. The methods described herein may be stored on a computer readable medium.

**DOSE CONTROL.** A vaporizer and/or vaporizer system may include dose control and/or dose metering. In general, dose control is described in U.S. patent application no. 14/960,259, filed on December 4, 2015, and herein incorporated by reference in its entirety.

As described above, a vaporizer and/or a device that is part of a vaporizer system as defined above may include a user interface (e.g., including an app or application software) that may be executed on a device in communication, which may be configured to determine, display, enforce and/or meter dosing. For example, a vaporizer may have a "unit dose" mode/indicator that is displayed on the vaporizer and/or an application. The unit dose could be changed by the connected application and/or by directly controlling the vaporizer. For example, a user may want to go from 1mg nicotine per dose to 2mg of nicotine per dose.

The dose unit may be programmable. For example, a user may program a dose based on previous (recorded) use; e.g., the user may press a "start" button on the app, take enough puffs until satisfied, and then press "stop" on the app. In addition, the user may input user-specific data that may be helpful in determining and/or metering dosing. For example, the user may input body weight, gender, and any other relevant data. Such info can be used for adjusting dose of therapeutic drugs such as pain killer, sleep aid, etc. accordingly.

As mentioned, in some implementations of the current subject matter, the vaporizer and/or app running on a device that is connected (or connectable) to the vaporizer may record use or operation of the device and may play back this use later. In general, the vaporizer or app may record a first operational parameter (e.g., temperature setting, ramp time to heat, etc.) and a second use parameter (e.g., number of puffs, cumulative dose, use time, etc.), may store the recorded operational parameter and use parameter as a use profile, may associate the recorded use profile with a control, button, icon, etc., and may program the device operation based on the use profile, so that the operational parameter is modified automatically as the actual operational parameter tracks with the recorded operational parameter.

For example, the user may record a use profile including the number of puffs (e.g., draw events, inhalations, etc.) between changes in the temperature, as well as the temperature so that this use profile may be replayed later, e.g., by selecting a button or other indicator associated with the recorded/programmed use profile. In some implementations, the vaporizer and/or app may record the temperature and one or more second use parameters, such as one or more of: puff time (duration), puff count (number of puffs), energy applied to vaporizable material (e.g., cumulative joules of energy), dosage/exposure, etc. Playback may be indexed on any of the recorded use parameters such as the number of puffs, cumulative duration of puffing, cumulative energy applied, cumulative dose, etc. and may set or modify the operational parameter (e.g., applied vaporization temperature, energy applied, etc.) of the vaporizer to the recorded temperature to match the recorded and/or programmed temperature as the vaporizer is operated, so that the same use profile will be followed. For example, a user may record a use profile while operating the device at a first temperature (e.g., 150 °C) for 5 draws (puffs), then increasing the temperature to 180 °C for five more puffs, then increasing the temperature to 200 °C for 10 puffs. The recorded operational profile may be stored on the vaporizer, app, or some other connected memory, and associated with a control (e.g., icon, graphic, text, button, etc.) on the vaporizer, app and/or a remote processor or memory. The recorded operational profile may then be played back, e.g., by selecting an icon (or button, control, text, etc.) on the app or vaporizer that has been associated with the recorded/programmed profile. During playback, the vaporizer may wait until the same or a similar operational parameter (e.g., puffs, time of use, applied power, dose, etc.) is matched or exceeded and may control the heater based on the recorded profile. In the example above, the recorded operational profile may be played back later by pressing the icon; the vaporizer and/or app may compare the use parameter (number of puffs, etc.) to the current operation of the vaporizer and may adjust the operational parameter accordingly to match the use profile.

The use profile may be recorded, or it may be programmed, or both (e.g., a recorded use profile may be modified by a user on the vaporizer and/or app, etc.).

In some examples, dose (e.g., cumulative dose) may be the use parameter that is monitored. In some implementations of the current subject matter, dose may be calculated as described in U.S. patent application no. 14/960,259, filed on 12/4/2015, previously incorporated by reference in its entirety. The cumulative dose may be stored for transmission and/or display. Further, the dose may be used to control operation of the vaporizer.

In one example of a nicotine dose control, the user could set a target cap for how much nicotine he/she wants in a day. In some implementations, the device won't lock the user out from having more, but it will notify if a target has been exceeded. Alternatively, the device may lock the user out.

**MONITORING - HEALTH and CESSATION.** A vaporizer and/or applications running on a device that is part of a vaporizer system consistent with implementations of the current subject matter may also be configured to monitor usage for a digital health regimen, and/or smoking cessation, etc. For example, similar to weight loss monitoring devices, a vaporizer or an app or both may be useful for people who want to reduce nicotine consumption, and/or keep track of how much nicotine consumed within a certain amount of time. For example, the vaporizer and/or app may be configured to allow cigarette-e-cigarette dual users to log in how many cigarettes they consume and compare the total amount of HPHCs (Harmful and Potentially Harmful Constituents) and nicotine they get on different days when they use different combinations.

The app and/or vaporizer may also provide additional motivation by providing messaging such as reporting how much of X compound is consumed, and may show how much money the former smoker is saving by reducing or eliminating smoking. This may be most relevant for nicotine, although it may be used for other substances as well. In some implementations, the user may enter their usual price per pack of cigarettes, which may be used as the baseline.

In some implementations of the current subject matter, the app may also allow a user to log other health related activities, such as from a fitness app, and/or may suggest correlations between nicotine usage and alcohol consumption, heart rate, blood pressure, workout time or weight changes, etc. For example, a user may enter a preferred unit dose (using presets, or estimated/recorded/programmable data as described above), and a dosage interval or total daily target. The vaporizer and/or app may then lock out after each dosage, and an alert may pop up on a user computing device (e.g., phone, smartwatch, tablet, etc.) when it's time for a next dosage, and the vaporizer automatically unlocks for this next dosage. This could be used as a user-elected reduction approach (step-down or cessation), or to maintain a prescribed therapeutic regimen (e.g., X mg of agent every Y hours, not to exceed Z mg / day).

In some implementations, the vaporizer and/or an affiliated app may have a dashboard style user-interface, in which users can log on and tabulate their progress over time. Data may be based on individual and/or group data. For example, the group data can show as a population of what the mean smoking-vaping switch rate is at any given time since starting to use a vaporizer. The apparatus may provide a view in which the user can select other users to define a group (cohort) based on their starting conditions: e.g., packs per day, age, gender, etc.

**USER PREFERENCES.** In some implementations, the vaporizer and/or an affiliated app may be customized based on user preferences, and may provide alerts or reminders. For example, in some implementations, the apparatus may save preferences for cartridges (e.g., "pods") of different flavors, strains, and/or strengths that may be preferred by the user. The app and/or vaporizer may save preferences for different use cases (e.g., 'going for a hike', 'bedtime', etc.).

In conjunction with cartridge sensing (as described above), in any of the implementations described herein, the vaporizer and/or app may also or alternatively suggest one or more use profiles (e.g., heating profiles). For example, based on the type of cartridge and/or based on user input on the type of vaporizable material (flavor, strain, strength, etc.) even in implementations not including cartridge detection, the vaporizer and/or app may suggest a use profile (e.g., "Other users enjoy this strain with profile X", or "Other users enjoy this strain at an initial temperature of 155°C").

**VAPORIZER SESSIONING.** A vaporizer and/or vaporizer system may include "session" control and/or session metering. In some aspects, a user may find it desirable to monitor and/or control consumption of the vaporizable material, not only for unit dosage described herein, but also for consumption over a certain time period, such as a day, week, month, one or more sessions, and/or the like. Such monitoring and/or control can beneficially allow a user to adjust an amount of vaporizable material available over a certain time period or session, a total time allowed for using the vaporizer, a time period between vaporizer sessions, other consumption settings to meet the needs or goals of the user, and/or the like. In an example of monitoring and/or controlling consumption of the vaporizable material for consumption over a certain time period, one or more sessions, and/or the like, it may be desirable for the application to provide a suggested dosage limit per period of time so that the user can continue to meet their desired dosage goals. The suggested dosage limit can be based on user data provided through the user interface, such as previous cigarette or vaping consumption over a set period of time. In some implementations, the user may provide their own dosage limit. In some implementations, for example, if a maximum level of consumption over a certain time period has been met, the device can lock the user out for a sufficient period of time to allow the user to experience a full session during the next use of the device and notify the user when a target has been exceeded.

In some aspects, the vaporizer may be configured to receive an indication of and/or determine a start of a vaporizer session. In some implementations, the indication of the start can include a user selection on the vaporizer application, a user selection of an input on the vaporizer and/or a device that is part of a vaporizer system, a user beginning an inhalation or puff, a user turning on the vaporizer, a user-defined or pre-programmed gesture, or any other user action or device input indicating the start of a vaporizer session.

As described above, a vaporizer and/or a device that is part of a vaporizer system as defined above may provide feedback or an indication to the user to signify a status or completion of a session. For example, feedback provided to the user can be any form of sensory feedback, such as for example visual feedback, auditory feedback, or tactile feedback. In some aspects, the feedback/indication can comprise one or more device cues that indicate the progress, status, and/or completion of a vaporizer session. The device cues can include one or more haptic cues, one or more visual cues, one or more audio cues, one or more vapor cues, one or more airflow cues, one or more mechanical cues, any other indication, and/or any combination of the above.

In some embodiments, a vaporizer and/or a device that is part of a vaporizer system may provide feedback or an indication to the user that a cigarette's worth of nicotine has been delivered.

For example, haptic cues can include a vibration or a vibration pattern. In some implementations, the haptic cues can indicate a status of a vaporizer session. For example, the vaporizer and/or a device that is part of a vaporizer system, via an output (e.g., output 115), can provide a first haptic cue to indicate the beginning of a vaporizer session. In some aspects, the first haptic cue may be a single vibration pulse, a predefined vibration pattern, or a user defined vibration or other haptic indication.

After the start of a vaporizer session, the vaporizer and/or a device that is part of a vaporizer system can provide one or more second haptic cues to indicate the progress of a vaporizer session. In some aspects, the second haptic cue can include vibration pulses of increasing or decreasing duration, intensity, and/or frequency. For example, the vaporizer may output a single vibration pulse to indicate a start of a vaporizer session, two vibration pulses to indicate a halfway point of the vaporizer session, and three vibration pulses to indicate the near completion or completion of the vaporizer session. In another example, the vaporizer may output a vibration pulse at a first intensity to indicate a start of a vaporizer session. The vaporizer may output the vibration pulse at a second intensity to indicate a halfway point of the vaporizer session, the second intensity higher than the first intensity. The vaporizer may also output the vibration pulse at a third intensity to indicate the completion or near completion of the vaporizer session, the third intensity higher than the second intensity. In another example, the vaporizer may output a vibration pulse for a first duration to indicate a start of a vaporizer session. The vaporizer may output the vibration pulse for a second duration to indicate a halfway point of the vaporizer session, the second duration longer than the first duration. The vaporizer may also output the vibration pulse for a third duration to indicate the completion or near completion of the vaporizer session, the third duration longer than the second duration.

Second haptic cues can also include vibration patterns to indicate the progress of a vaporizer session. For example, the vaporizer may provide a first vibration pattern to indicate a start of a vaporizer session, a second vibration pattern to indicate a halfway point of the vaporizer session, and a third vibration pattern to indicate the completion or near completion of the vaporizer session.

In some aspects, the second haptic cue may be the same as the first haptic cue provided at a later time. In some implementations, the vibration or haptic cue may be provided by a linear resonant actuator (LRA) or an eccentric rotating mass (ERM) motor. Though the vaporizer sessions are described in three segments above, a vaporizer session can be partitioned into fewer or more segments consistent with implementations of the current subject matter.

Any of the above examples may also be used in combination to indicate a status or progress of a vaporizer session. For example, as described above, the vaporizer may output a single vibration pulse to indicate a start of a vaporizer session, two vibration pulses to indicate a halfway point of the vaporizer session, and three vibration pulses to indicate the near completion of the vaporizer session. To indicate the completion of the vaporizer session, the vaporizer may output a predefined or user defined vibration pattern. In some aspects, the vaporizer may also include a countdown to indicate the completion or near completion of a vaporizer session. For example, the vaporizer and/or a device that is part of a vaporizer system may determine that there are three unit dosages remaining. The vaporizer may output three vibration pulses to indicate the remaining dosages. After the user completes one dose, the vaporizer may then output two vibration pulses to indicate the remaining doses. After the user completes another dose, the vaporizer may then output one vibration pulse to indicate the remaining dose. At the completion of the last dose, the vaporizer may indicate the completion by failing to vibrate again, by outputting a long duration vibration, by outputting a vibration pattern, by outputting a series of vibration pulses, or by outputting any other haptic indication.

As described above, the vaporizer and/or a device that is part of a vaporizer system can provide visual cues to a user to indicate a status of a vaporizer session. In some implementations, visual cues can include a light or light pattern to indicate the start, the progress/status, and/or the completion of a vaporizer session. For example, the vaporizer and/or a device that is part of a vaporizer system, via an output (e.g., output 115), can provide a first visual cue to indicate the beginning of a vaporizer session. The output 115 can include LED lights, a user display, or other light displays. In some aspects, the first visual cue may be a single light flash, a light array, a light color, a light brightness, a predefined light pattern, or a user defined light pattern, or any other visual indication.

After the start of a vaporizer session, the vaporizer and/or a device that is part of a vaporizer system can provide one or more second visual cues to indicate the progress of a vaporizer session. In some implementations, determining when to provide the one or more second visual cues can be based on monitoring a usage of the vaporizer device. For example, the monitoring can include determining an amount of vaporizable material consumed or remaining in a vaporizer session and providing feedback (e.g., second visual cue or other cue) to indicate a status or state of the vaporizer session. In some aspects, the second visual cue can include light displays of increasing or decreasing duration, intensity or brightness, number and/or frequency. The second visual cue may also include different colors, light patterns, arrays. For example, the vaporizer may output a light flash to indicate a start of a vaporizer session, two light flashes to indicate a halfway point of the vaporizer session, and three light flashes to indicate the near completion or completion of the vaporizer session. In another example, the vaporizer may output different colors to indicate a status/progress of a vaporizer session. In some aspects, the vaporizer may output a first color (e.g., green) to indicate a start of a vaporizer session. The vaporizer may output a second color (e.g., yellow) to indicate a halfway point of the vaporizer session. The vaporizer may also output a third color (e.g., red) to indicate the completion or near completion of the vaporizer session. In another example, the vaporizer may output a light flash at a first intensity/brightness level to indicate a start of a vaporizer session. The vaporizer may output the light flash at a second intensity/brightness level to indicate a halfway point of the vaporizer session, the second intensity/brightness level higher than the first intensity/brightness level. The vaporizer may also output the light flash at a third intensity/brightness level to indicate the completion or near completion of the vaporizer session, the third intensity/brightness level higher than the second intensity/brightness level.

In some implementations, the vaporizer may include a light array. The light array can be configured to provide different light arrays or light patterns to indicate the progress of a vaporizer session. For example, the vaporizer may include three LED lights as part of the outputs 115. In some implementations, a first LED light may turn on to indicate the start of a vaporizer session. As the session progresses, a second LED light may turn on in addition to, or in place of, the first LED light to indicate a halfway point of the vaporizer session. The vaporizer may also turn on a third LED light in addition to, or in place of, the first and/or second LED lights to indicate the completion or near completion of the vaporizer session. In some implementations, the sequential illumination of the LED lights in the manner described above can indicate the amount of vaporizable material dispensed relative to the desired total delivered dose over a period of time, as set by the user or recommended by the application. Though three lights are described above, more or fewer lights are possible. In another example, the vaporizer can provide a first light pattern to indicate a start of a vaporizer session, a second light pattern to indicate a halfway point of the vaporizer session, and a third light pattern to indicate the completion or near completion of the vaporizer session. The first, second, and third light patterns can include any combination of flashing lights, color, brightness, etc. In some aspects, the light patterns can be predefined or can be user defined.

In some aspects, the second visual cue may be the same as the first visual cue provided at a later time. Though the vaporizer sessions are described in three segments above, a vaporizer session can be partitioned into fewer or more segments consistent with implementations of the current subject matter.

Any of the above examples may also be used in combination to indicate a status or progress of a vaporizer session. For example, as described above, the vaporizer may output a single green light flash to indicate a start of a vaporizer session, two yellow light flashes to indicate a halfway point of the vaporizer session, and three red light flashes to indicate the near completion of the vaporizer session. To indicate the completion of the vaporizer session, the vaporizer may output a predefined or user defined light pattern. In some implementations, the light pattern includes a combination of colors, flashes, brightness levels, and different lights. In some aspects, the vaporizer may also include a countdown to indicate the completion or near completion of a vaporizer session. For example, the vaporizer and/or a device that is part of a vaporizer system may determine that there are three unit dosages remaining. The vaporizer may output three light flashes to indicate the remaining dosages. After the user completes one dose, the vaporizer may then output two light flashes to indicate the remaining doses. After the user completes another dose, the vaporizer may then output one light flash to indicate the remaining dose. At the completion of the last dose, the vaporizer may indicate the completion by failing to turn on a light, by outputting a long duration light flash, by outputting a light pattern, by outputting a light color (e.g., red), by outputting a series of light flashes, or by outputting any other visual indication. In some aspects, one or more visual cues can be provided via an application on a user interface such as a smart phone screen, tablet, or other computing apparatus. The user interface can also display at least a portion of the information retrieved from one or more sensors associated with the vaporizer. Such displaying of information associated with the usage of the vaporizer may allow for improved monitoring of a vaporizer session and/or vaporizer usage/operation.

As described above, the vaporizer and/or a device that is part of a vaporizer system can provide audio cues to a user to indicate a status of a vaporizer session. In some implementations, audio cues can include a sound or sound pattern to indicate the start, the progress/status, and/or the completion of a vaporizer session. For example, the vaporizer and/or a device that is part of a vaporizer system, via an output (e.g., output 115), can provide a first audio cue to indicate the beginning of a vaporizer session. The output 115 can include a speaker or an audio control. In some aspects, the first audio cue may be a single audio sound (e.g., beep), a plurality of sounds, a song, an audio volume, a predefined audio pattern, or a user defined audio pattern, or any other audio indication. In some implementations, the vaporizer and/or a device that is part of a vaporizer system can emit a sound as part of normal operation. In some aspects, the vaporizer and/or a device that is part of a vaporizer system can alter, imitate, or emit the sound to indicate the start of the vaporizer session. For example, the vaporizer can emit a crackling sound similar to when a user inhales the vaporized material to indicate the start of the vaporized material.

After the start of a vaporizer session, the vaporizer and/or a device that is part of a vaporizer system can provide one or more second audio cues to indicate the progress of a vaporizer session. In some aspects, the second audio cue can include audio sounds of increasing or decreasing duration, volume, number, and/or frequency. The second audio cue may also include sounds having different patterns or songs. For example, the vaporizer may output a single beep to indicate a start of a vaporizer session, two beeps to indicate a halfway point of the vaporizer session, and three beeps to indicate the near completion or completion of the vaporizer session. In another example, the vaporizer may output different sounds to indicate a status/progress of a vaporizer session. In some aspects, the vaporizer may output a first sound (e.g., crackling sound) to indicate a start of a vaporizer session. The vaporizer may output a second sound (e.g., beep) to indicate a halfway point of the vaporizer session. The vaporizer may also output a third sound (e.g., click) to indicate the completion or near completion of the vaporizer session. In another example, the vaporizer may output a sound (e.g., crackling sound) at a first volume level to indicate a start of a vaporizer session. The vaporizer may output the sound at a second volume level to indicate a halfway point of the vaporizer session, the second volume level lower than the first volume level. The vaporizer may also output the sound at a third volume level to indicate the completion or near completion of the vaporizer session, the third volume level lower than the second volume level.

In some implementations, the vaporizer may alter a sound to indicate the progress of a vaporizer session. For example, the vaporizer may output a sound (e.g., crackling sound) to indicate the start of a vaporizer session. As the session progresses, the vaporizer may introduce a stutter to the sound to indicate a halfway point of the vaporizer session. In some aspects, the stutter can be implemented using a capacitor or oscillator that can control the frequency of the sound outputted. The vaporizer may increase the stutter on the sound, replicating a flame or cigarette sputtering out, to indicate the completion or near completion of the vaporizer session. In another example, the vaporizer can provide a first sound pattern to indicate a start of a vaporizer session, a second sound pattern to indicate a halfway point of the vaporizer session, and a third sound pattern to indicate the completion or near completion of the vaporizer session. The first, second, and third sound patterns can include any combination of tones, pitch, frequency, volume, melodies, etc. In some aspects, the sound patterns can be predefined or can be user defined.

In some aspects, the second audio cue may be the same as the first audio cue provided at a later time. Though the vaporizer sessions are described in three segments above, a vaporizer session can be partitioned into fewer or more segments consistent with implementations of the current subject matter.

Any of the above examples may also be used in combination to indicate a status or progress of a vaporizer session. For example, as described above, the vaporizer may output a single sound (e.g., crackling) to indicate a start of a vaporizer session, a second sound twice (e.g., two beeps) to indicate a halfway point of the vaporizer session, and a third sound (e.g., click) three times to indicate the near completion of the vaporizer session. To indicate the completion of the vaporizer session, the vaporizer may output a predefined or user defined sound pattern. In some implementations, the sound pattern includes a combination of tones, pitch, frequency, volume, melodies, etc. In some aspects, the vaporizer may also include a countdown to indicate the completion or near completion of a vaporizer session. For example, the vaporizer and/or a device that is part of a vaporizer system may determine that there are three unit dosages remaining. The vaporizer may output three sounds (e.g., beeps) to indicate the remaining dosages. After the user completes one dose, the vaporizer may then output two beeps to indicate the remaining doses. After the user completes another dose, the vaporizer may then output one beep to indicate the remaining dose. At the completion of the last dose, the vaporizer may indicate the completion by outputting a different sound (e.g., click), by outputting a long duration beep, by outputting a sound pattern, by outputting a higher volume sound (e.g., loud beep), by outputting a series of quick beeps, or by outputting any other audio indication.

As described above, the vaporizer and/or a device that is part of a vaporizer system can provide a change in vapor content/amount to a user to indicate a status of a vaporizer session. The change in vapor content/amount can comprise changes to vapor and/or aerosol strength, vapor and/or aerosol density, vapor and/or aerosol volume, vapor and/or aerosol flavor, vapor and/or aerosol temperature, and/or similar vapor and aerosol characteristics of the vapor or aerosol generated by the vaporizer. In some implementations, the vaporizer and/or a device that is part of a vaporizer system can provide an increase or decrease vapor volume to indicate the start, the progress/status, and/or the completion of a vaporizer session. For example, the vaporizer and/or a device that is part of a vaporizer system can provide a first vapor cue to indicate the beginning of a vaporizer session. In some aspects, the first vapor cue may be that the vaporizer and/or a device that is part of a vaporizer system provides a first volume of vapor. The first volume may be predetermined or may be programmed by the user. In some aspects, changing a volume of vapor can include changing a temperature set-point, to allow users to get less vapor. In some aspects, the first vapor cue may be a specific vapor strength, vapor density, vapor volume, vapor flavor, vapor temperature, or any other vapor characteristic.

After the start of a vaporizer session, the vaporizer and/or a device that is part of a vaporizer system can provide one or more second vapor cues to indicate the progress of a vaporizer session. In some aspects, the second vapor cue can include a change in the vapor and/or aerosol strength, vapor and/or aerosol density, vapor and/or aerosol volume, vapor and/or aerosol flavor, vapor and/or aerosol temperature, and/or similar vapor and aerosol characteristics of the vapor or aerosol generated by the vaporizer. For example, the vaporizer may output a first volume of vapor to indicate a start of a vaporizer session, a second volume of vapor to indicate a halfway point of the vaporizer session, and a third volume of vapor to indicate the near completion or completion of the vaporizer session. In some aspects, the second volume is greater than the first volume and the third volume is less than second and first volumes. In this way, a user may be able to correlate the volume of vapor exhaled with the remaining time or doses left in a session. In other aspects, the user may experience an increasing vapor volume to indicate the different stages. In another example, the vaporizer may output different flavors to indicate a status/progress of a vaporizer session. In some aspects, the vaporizer may output a first flavor (e.g., mint) to indicate a start of a vaporizer session. The vaporizer may output a second flavor (e.g., cherry) to indicate a halfway point of the vaporizer session. The vaporizer may also output a third flavor (e.g., menthol) to indicate the completion or near completion of the vaporizer session. In some aspects, the flavors indicating the different stages differ from a flavor chosen for the rest of the vaporizer session. In this way, the user may better identify the different vapor cues to monitor their progress.

In another example, the vaporizer may output vapor at a first temperature to indicate a start of a vaporizer session. The vaporizer may output the vapor at a second temperature to indicate a halfway point of the vaporizer session, the second temperature higher than the first temperature. The vaporizer may also output the vapor at a third temperature to indicate the completion or near completion of the vaporizer session, the third temperature higher than the second temperature. In another example, the vaporizer may output vapor at a first density to indicate a start of a vaporizer session. The vaporizer may output the vapor at a second density to indicate a halfway point of the vaporizer session, the second temperature higher than the first temperature. The vaporizer may also output the vapor at a third density to indicate the completion or near completion of the vaporizer session, the third temperature lower than the second temperature. In this example, the density can be lower at the beginning and end of the vaporizer session and greater at the halfway point to help the user determine the progress of the vaporizer session. However, as with any of the above examples, other configurations possible. For example, the vapor density may increase or decrease through vaporizer session to indicate progress of the vaporizer session.

In some aspects, the second vapor cue may be the same as the first vapor cue provided at a later time. Though the vaporizer sessions are described in three segments above, a vaporizer session can be partitioned into fewer or more segments consistent with implementations of the current subject matter.

Any of the above examples may also be used in combination to indicate a status or progress of a vaporizer session. For example, as described above, the vaporizer may provide a first flavor (e.g., mint) at a first temperature to indicate a start of a vaporizer session. The vaporizer may provide a second flavor (e.g., cherry) at a second temperature (e.g., higher than the first) to indicate a halfway point of the vaporizer session. The vaporizer may also provide a third flavor (e.g., menthol) at a third temperature (e.g., higher than the second) to indicate the near completion of the vaporizer session. To indicate the completion of the vaporizer session, the vaporizer may output a predefined or user defined vapor characteristic. In some implementations, the vapor characteristic includes any combination of volume, flavor, density, temperature, and strength. For example, a user may wish to have a high volume, high temperature, and high density peppermint flavor as its last dose which indicates the completion of the vaporizer session.

As described above, the vaporizer and/or a device that is part of a vaporizer system can provide airflow cues to a user to indicate a status of a vaporizer session. In some implementations, airflow cues can include a change in airflow through a mouthpiece of the vaporizer (e.g., mouthpiece 244) to indicate the start, the progress/status, and/or the completion of a vaporizer session. For example, the vaporizer and/or a device that is part of a vaporizer system can provide a first airflow cue to indicate the beginning of a vaporizer session. In some aspects, the first airflow cue may be a level of airflow through the mouthpiece, a change in airflow through the mouthpiece, a pattern of airflow through the mouthpiece, or any other airflow indication.

After the start of a vaporizer session, the vaporizer and/or a device that is part of a vaporizer system can provide one or more second airflow cues to indicate the progress of a vaporizer session. In some aspects, the second airflow cue can include airflows of increasing or decreasing, resistance and/or duration. For example, the vaporizer may provide a first resistance to airflow of the mouthpiece to indicate a start of a vaporizer session. As the vaporizer session progresses, the vaporizer may provide a second level of resistance to airflow of the mouthpiece to indicate a halfway point of the vaporizer session. The second resistance higher than the first resistance to make it harder for the user to inhale through the mouthpiece. The vaporizer may also provide a third resistance to airflow of the mouthpiece to indicate the completion or near completion of the vaporizer session, the third resistance higher than the second resistance to make it harder for the user to inhale through the mouthpiece. In some implementations, the vaporizer and/or a device that is part of a vaporizer system can completely restrict airflow through the mouthpiece to indicate the completion of the vaporizer session. In another example, the vaporizer may provide a first airflow pattern to indicate a start of a vaporizer session. For example, the first airflow pattern may comprise a single brief partial or complete blocking of airflow through the mouthpiece. The vaporizer may provide a second airflow pattern to indicate a halfway point of the vaporizer session. The second airflow pattern can comprise two brief partial or complete blockings of airflow through the mouthpiece. The vaporizer may also provide a third airflow pattern to indicate the completion or near completion of the vaporizer session. In some aspects, the third airflow pattern can comprise three brief partial or complete blockings of airflow through the mouthpiece. In some implementations, the first, second, and third airflow patterns can include any combination of airflow resistance and/or blocking to the mouthpiece.

In some aspects, the second airflow cue may be the same as the first airflow cue provided at a later time. Though the vaporizer sessions are described in three segments above, a vaporizer session can be partitioned into fewer or more segments consistent with implementations of the current subject matter.

Any of the above examples may also be used in combination to indicate a status or progress of a vaporizer session. For example, as described above, the vaporizer may provide a first airflow pattern (e.g., one partial or complete blocking) at a first resistance to indicate a beginning of a vaporizer session. As the vaporizer session progresses, the vaporizer may provide a second airflow pattern (e.g., two partial or complete blockings) at a second resistance to indicate a halfway point of the vaporizer session. The second resistance higher than the first resistance to make it harder for the user to inhale through the mouthpiece. The vaporizer may also provide a third airflow pattern (e.g., three partial or complete blockings) at a third resistance to indicate the near completion of the vaporizer session. The third resistance higher than the second resistance to make it harder for the user to inhale through the mouthpiece.

In some implementations, to indicate the completion of the vaporizer session, the vaporizer may provide a predefined or user defined airflow pattern. In some implementations, the airflow pattern can include any combination of airflow resistance and/or blocking to the mouthpiece. In some aspects, the vaporizer may also include a countdown to indicate the completion or near completion of a vaporizer session. For example, the vaporizer and/or a device that is part of a vaporizer system may determine that there are three unit dosages remaining. The vaporizer may provide three partial or complete blockings to airflow through the mouthpiece to indicate the remaining dosages. After the user completes one dose, the vaporizer may then provide two partial or complete blockings to airflow through the mouthpiece to indicate the remaining doses. After the user completes another dose, the vaporizer may then provide one partial or complete blocking to airflow through the mouthpiece to indicate the remaining dose. At the completion of the last dose, the vaporizer may indicate the completion by completely blocking airflow through the mouthpiece or by providing any other airflow indication. In another example, the countdown may comprise higher levels of resistance to airflow as the user approaches the end of the vaporizer session.

As described above, the vaporizer and/or a device that is part of a vaporizer system can provide mechanical cues to a user to indicate a status of a vaporizer session. In some implementations, mechanical cues can include a mechanical change to at least a portion of the vaporizer to indicate the start, the progress/status, and/or the completion of a vaporizer session. For example, the vaporizer and/or a device that is part of a vaporizer system, via an output (e.g., output 115), can provide a first mechanical cue to indicate the beginning of a vaporizer session. The output 115 can include a spring loaded tab, a portion of the vaporizer that extends or contracts, or any other mechanical change to the vaporizer that a user can see and/or feel. In some aspects, the first mechanical cue may be a tab that extends from the vaporizer, a portion of the vaporizer that retracts within the body of the vaporizer, a portion of the vaporizer that rotates, or any other mechanical indication.

After the start of a vaporizer session, the vaporizer and/or a device that is part of a vaporizer system can provide one or more second mechanical cues to indicate the progress of a vaporizer session. In some aspects, the second mechanical cue can include mechanical changes of increasing or decreasing duration, number and/or frequency. For example, the vaporizer may extend a single tab or portion to indicate a start of a vaporizer session, two tab/portions to indicate a halfway point of the vaporizer session, and three tab/portions to indicate the near completion or completion of the vaporizer session. In another example, the vaporizer may output different mechanical changes to indicate a status/progress of a vaporizer session. In some aspects, the vaporizer may output a first mechanical change (e.g., extend a tab) to indicate a start of a vaporizer session. The vaporizer may output a second mechanical change (e.g., retraction of a portion) to indicate a halfway point of the vaporizer session. The vaporizer may also output a third mechanical change (e.g., rotation of a portion) to indicate the completion or near completion of the vaporizer session. In another example, the vaporizer may output a mechanical change (extension of a portion) for a first duration to indicate a start of a vaporizer session. The vaporizer may output the mechanical change for a second duration to indicate a halfway point of the vaporizer session, the second duration longer than the first duration. The vaporizer may also output the mechanical change for a third duration to indicate the completion or near completion of the vaporizer session, the third duration longer than the second duration.

In another example, the vaporizer can provide a first mechanical pattern to indicate a start of a vaporizer session, a second mechanical pattern to indicate a halfway point of the vaporizer session, and a third mechanical pattern to indicate the completion or near completion of the vaporizer session. The first, second, and third light patterns can include any combination of extending, retracting, rotating, or otherwise mechanically changing any portion or portions of the vaporizer. In some aspects, the mechanical patterns can be predefined or can be user defined.

In some aspects, the second mechanical cue may be the same as the first mechanical cue provided at a later time. Though the vaporizer sessions are described in three segments above, a vaporizer session can be partitioned into fewer or more segments consistent with implementations of the current subject matter.

Any of the above examples may also be used in combination to indicate a status or progress of a vaporizer session. For example, as described above, the vaporizer may extend a single tab and rotate a portion to indicate a start of a vaporizer session. The vaporizer may also extend two tabs and rotate a second portion of the vaporizer to indicate a halfway point of the vaporizer session, and extend three tabs and rotate a third portion of the vaporizer to indicate the near completion of the vaporizer session. To indicate the completion of the vaporizer session, the vaporizer may output a predefined or user defined mechanical pattern. In some implementations, the light pattern includes any combination of extending, retracting, rotating, or otherwise mechanically changing any portion or portions of the vaporizer. In some aspects, the vaporizer may also include a countdown to indicate the completion or near completion of a vaporizer session. For example, the vaporizer and/or a device that is part of a vaporizer system may determine that there are three unit dosages remaining. The vaporizer may extend three tabs to indicate the remaining dosages. After the user completes one dose, the vaporizer may then retract one tab and keep two tabs extended to indicate the remaining doses. After the user completes another dose, the vaporizer may then retract another tab and keep one tab extended to indicate the remaining dose. At the completion of the last dose, the vaporizer may indicate the completion by retracting the last tab, by rotating a portion of the vaporizer (e.g., mouthpiece), by outputting a mechanical pattern, by extending the three tabs again, by extending and retracting the tabs a number of times, or by outputting any other mechanical indication.

As stated above, the vaporizer and/or a device that is part of a vaporizer system can use any of the haptic cues, visual cues, audio cues, vapor cues, airflow cues, or mechanical cues discussed above or any other indication in combination to indicate a status or progress of a vaporizer session. For example, the vaporizer can provide a single audio beep along with a single vibration pulse and a single light flash to indicate the beginning of a vaporizer session. The vaporizer can also provide two audio beeps along with two vibration pulses and two light flashes to indicate a halfway point of the vaporizer session. The vaporizer can also provide three audio beeps along with three vibration pulses and three light flashes to indicate the completion or near completion of the vaporizer session. In another example, the vaporizer can provide a first vapor volume along with a first airflow resistance and a first mechanical change (e.g., portion extension) to indicate a beginning of a vaporizer session. The vaporizer can also provide a second vapor volume along with a second airflow resistance and a second mechanical change (e.g., portion rotation) to indicate a halfway point of the vaporizer session. In some aspects, the second vapor volume is greater than the first vapor volume and the second airflow resistance is greater than the first airflow resistance. The vaporizer can also provide a third vapor volume along with a third airflow resistance and a third mechanical change (e.g., portion retraction) to indicate the completion or near completion of the vaporizer session. In some aspects, the third vapor volume is greater than the second and first vapor volume and the third airflow resistance is greater than the second and first airflow resistance. In some implementations, in order to indicate completion of the vaporizer session, the vaporizer may provide an audio sound (e.g., click) along with a long duration vibration pulse and a light pattern. Additionally, the vaporizer may completely block airflow through the mouthpiece and provide a mechanical change (e.g., portion extension) until a new session is started. While a few examples are described above for illustrative purposes, any other combination of cues is possible to indicate different segments and progress of a vaporizer session.

**NEW SESSION INITIATION.** After completion of a vaporizer session, it may be desirable to control the start of a new session. In some aspects, the user may wish to limit or monitor the vaporizer session for a given time period (e.g., sessions per hour, day, week, month, etc.). In order to clearly distinguish vaporizer sessions from one another, the vaporizer and/or a device that is part of a vaporizer system can require a specific user input, wait time between sessions, device setting or status, or other criteria before starting a new vaporizer session.

In some aspects, the vaporizer may include a motion, pressure, or other sensor configured to receive input from a user. The vaporizer and/or a device that is part of a vaporizer system can receive data from one or more of the sensors to monitor usage and/or operation of the vaporizer. The vaporizer may require a specific user action or input before it allows a new session to take place. For example, the specific user action may include tapping the vaporizer or a portion of the vaporizer on a surface (e.g., table) one or more times. In another example, the specific user action may include pressing a button on the vaporizer. Also, a user may tap the vaporizer or a portion of the vaporizer to indicate that the user wishes to start a new session. In some aspects, pulling a charging case or battery out of the vaporizer body may allow for a new session to take place. Additionally, removal and/or replacement of a cartridge can initiate a new session. In some implementations, a new session may can be initiated through a connection with an application on a mobile device or computer. In some aspects, the vaporizer may connect with the application over any wired or wireless network. For example, the connection may be through Bluetooth, near-field communication (NFC), WiFi, ultrasound, ZigBee, RFID, cellular, USB, Ethernet, or any other communication means. In some implementations, the application is displayed on a user interface to receive inputs from a user. The user may select to start a new session through the application. Additionally, the user may configure settings relating to the start of a new vaporizer session. For example, the user may set a minimum wait time between sessions, a defined action/gesture needed to start a new session (e.g., tap the vaporizer), or a specific condition of the vaporizer (e.g., oven temperature) before starting a new session.

**SOFTWARE APPLICATION FUNCTIONALITY.** As stated above, the vaporizer and/or a device that is part of a vaporizer system can include a user interface (e.g., including an app or application software) that may be executed on a device in communication, which may be configured to determine, display, enforce and/or meter session dosage or sessioning. For example, a vaporizer may have a "session dose" mode/indicator that is displayed on the vaporizer and/or an application. The session dose could be changed by the connected application and/or by directly controlling the vaporizer. For example, a user may want to set a daily nicotine dosage (e.g., 20 mg per day) and gradually decrease that daily dosage over time (e.g., to 10 mg per day). A user may partition the daily dosage into a number of vaporizer sessions allowed per day. For example, the user may set the number of allowed sessions per day to four sessions and set the session dosage to 5 mg of nicotine. The user may then keep the number of vaporizer sessions at four but gradually decrease the session dosage of nicotine to 2.5 mg to help reduce overall nicotine consumption. In some embodiments, the user interface may collect various forms of user data for use in deriving a recommended session dosage or sessioning. For example, the user interface may collect information pertaining to the user's preferred cigarette type or cigarette manufacturer, and/or the typical number of preferred cigarettes smoked in one day. In some embodiments, the application may, through the user interface, provide a suggested dosage limit per period of time (e.g., day, week, etc.) so that the user continues to meet their desired dosage goals.

In other aspects, the user may want to adjust other configurations of a vaporizer session. For example, in addition to setting a minimum wait time between sessions, the user may also configure blackout periods where the user is unable to start a session (e.g., during the morning or before a regular meeting). Additionally, the user may also set overall nicotine thresholds for a certain time period (e.g., hour, day, week, month, year, etc.) where the vaporizer will not function if the user satisfies the threshold. Additionally, the user may also set certain override procedures to override previously defined rules/thresholds. For example, the user may want to share their device or simply consume more vaporizer material than allowed based on previous settings. The user may enter in a passcode, answer a series of questions, provide other authentication, or otherwise confirm that they wish to override the previous settings. The user may even set no minimum wait time between sessions.

It also may be desirable for the user interface to provide a visualization of a status of a vaporizer session, daily dosage, or other vapor setting. For example, the user interface may provide a visualization of a virtual pack of cigarettes comprising 20 cigarettes. The virtual pack of cigarettes may correspond to a vaporizer session dosage, daily dosage, or other defined dosage. As the user progresses through a session, the number of cigarettes in the virtual pack of cigarettes may disappear, change color, or otherwise indicate a completion of a portion (1/20^{th}) of the session or other dosage. While the above example uses a pack of 20 cigarettes, other numbers of cigarettes and other indications are possible. For example, the number of cigarettes can correspond to the number of vaporizer sessions remaining for the day, week, month, year, etc. Additionally, the visualization may be matches, cigars, lighters, or any other visualization.

**DEVICE CONTROL AND CUSTOMIZATION.** As mentioned above, the vaporizer may be controlled in part by user input to an affiliated app. For example, particular aspects of the vaporizer that may be controlled may include changing a temperature set-point, for example to allows users to get less vapor if they need to be less conspicuous. This may also allow the user to reduce harshness and active ingredient consumption per puff.

The app may also provide a more precise indication of battery level beyond what is displayed on the vaporizer. For example, during charging, the app may indicate time remaining.

As mentioned above, the app may also provide firmware updates to the vaporizer.

The affiliated (connected) app may also allow the user to switch between nicotine and other vaporizable material modes, which may likely have different temperature set points, for devices that accept multiple types of vaporizable materials.

A vaporizer and/or a device that is part of a vaporizer system may use received signal strength indicator (rssi) to help a user locate a lost vaporizer. In addition, the app may allow the user to cause the vaporizer to vibrate, flash and/or emit sound(s) as an alarm, including for helping to locate a misplaced apparatus. For example, a temperature change, vibration or flash lights may also be the indicator of whether the vaporizer is hiding nearby. In some implementations, the vaporizer may also help locate a misplaced phone when connected via changing LED colors depending on the distance between the vaporizer and the phone.

A vaporizer and/or an app may be used to adjust LED brightness and color of the vaporizer. For example, for vaporizers with multiple LEDs, a user may download personalized indicator patterns to the device. In addition to making the vaporizer feel more personalized, this may have enhanced utility as it may make it easy to identify which vaporizer belongs to a particular owner.

In some implementations, the temperature of the vaporizer may be adjusted by using a graphical user interface that allows both gross and precise control of the vaporizer temperature with a single finger. For example, a graphical user interface (GUI) may include a display of the temperature visually indicating the current temperature and/or target temperature of the vaporizer; this temperature may be adjusted up or down (within a range). In this example, to adjust the temperature, the user may hold a fingertip in a location on or against the indicator, causing indicators to appear on either side of the temperature when the vaporizing temperature may be adjusted up (on right side) or down (on left side). Quickly sliding a finger over the adjacent indicators may rapidly move the temperature setting in large intervals (e.g., by 3 degree, 5 degree, 10 degree, 15 degree, 20 degrees, 25 degrees, 30 degrees, 35 degrees, etc., intervals). Large interval adjustment is indicated by the large circles. Holding a fingertip on the temperature indicator or adjacent indicators for a predetermined longer period of time (e.g., 1 second, 2 seconds, 3 seconds, 4 second, 5 seconds, etc.) may open a fine temperature control; moving the figure along the fine temperature control may allow increasing/decreasing the selected temperature by fine amounts (e.g., 0.1 degrees, 0.5 degrees, 1 degree, 2 degrees, etc.). The temperature change is shown in the central temperature indicator.

**SELF-CLEANING.** A vaporizer may be configured to include a self-cleaning mode, in which the vaporizer is configured to operate the heater at a predetermined high temperature (e.g., >= 600°F) for a self-cleaning time (e.g., greater than 1 min, greater than 2 min, greater than 3 min, greater than 4 min, greater than 5 min, greater than 6 min, greater than 7 min, greater than 8 min, greater than 9 min, greater than 10 min, greater than 12 min, greater than 15 min, etc.; or between 1 min and 20 min, between 1 min and 15 min, between 1 min and 10 min, etc.). The self-cleaning mode may be operated directly by the vaporizer, or it may be operated in conjunction with an application (app) or the like.

A self-cleaning mode may be operated in conjunction with an accelerometer or other sensor(s) of a vaporizer. For example, the accelerometer may be used to determine if the vaporizer is not held or carried by the user before entering the self-cleaning operation. For example, self-cleaning may be permitted only when the device has been "still" (e.g., set or held on a resting surface) for a predetermined time period, such as 30 seconds, 1 min, 1.5 min, 2 min, 2.5 min, 3 min, etc. The self-cleaning mode may also only be permitted in implementations (such as shown in FIGS. 2A-2C) having an oven or heating chamber door when the door is secured over the device.

The self-cleaning mode may also be terminated, and the device allowed to cool if the device is picked up or moved (e.g., based on accelerometer input). During self-cleaning, the device may provide a visual, audible or tactile output indicating that self-cleaning is underway. For example, one or more indicators may illuminate or flash (e.g., Red, red and blue, white, etc.) to indicate self-cleaning is operating. In some implementations, the vaporizer may also or alternatively indicate self-heating by emitting a tone, beep, or whine, or the like.

**ANTI-THEFT, PARENTAL LOCK, AND CHILD-PROOFING.** Any of the devices described herein may include a device lock, as mentioned above. For example, the app and or vaporizer may authenticate to a mobile device using encryption, as an anti-counterfeit mechanism. A similar scheme may be used to tie the vaporizer to the owner's mobile communications device (e.g., phone, smartwatch, pad, etc.), such that if stolen the device is disabled to prevent others from using it. In some implementations, the vaporizer may connect periodically to the mobile communications device to verify. In some implementations, a user may connect to the application on the user's mobile device or computer and provide authentication to enable operation of the vaporizer. In some aspects, the authentication includes password or PIN entry, a defined gesture (e.g., tap three times), selection of a confirmation button, a voice authentication, or a biometric authentication (e.g., facial recognition) inputted into the application.

The vaporizers described herein may also include parental lockout (e.g., child-proofing). For example, a device could be 'locked' for parents who want to make sure their children won't use the device. For parental lockout, in addition to Bluetooth or other relatively long range communications, the apparatus may also implement a near-field communications (NFC) tag on the vaporizer. NFC readers are built into many smartphones. One feature of NFC is that it only works in very short range. This would make unlocking very easy - you just tap the phone against the vaporizer. NFC tags are extremely cheap and small and may be used in addition to, or instead of, other wireless communication modes, such as Bluetooth. NFC could be used to implement some of the other features described above.

In some implementations, authentication takes place at the vaporizer. For example, the vaporizer may include a thumbprint sensor which enables operation of the vaporizer with a valid thumbprint. In another example, the vaporizer may require a specific action/gesture to enable operation. The specific action or gesture may include a password or PIN entry, a defined gesture (e.g., tap three times), a voice authentication, selection of a confirmation button, or a biometric authentication (e.g., facial recognition).

**GPS FOR LOCATOR, ORDERING, AND SOCIAL NETWORKING.** Any of the apparatuses described herein (e.g., vaporizers and/or an affiliated app) may include location services (GPS).

For example, a user buying cartridges for the vaporizer directly from a source may use an app to understand exactly how many cartridges that the user has and how many they have left. A retailer may use this information to offer the user to auto-order more when they are running low.

In any of the apparatuses described herein, the app and/or the vaporizer may include a GPS or may communicate with a GPS to determine location of the vaporizer. Locational information may be used to tell a user the closest retailer to buy more cartridges, to use location service for delivery, to order through smart phone (e.g., usage tracker combined with auto-refill), and/or to inform the user of relevant local legislation about e-cig use.

In addition, any of the vaporizers and apps described herein may be used to enhance the social experience of the user, including for interaction with other users, and communication with a particular user.

In some implementations, the vaporizer and/or app may profile users and tell them how they compare to others. For example, the vaporizer and/or app may indicate what percentile a user's nicotine consumption fall into and/or may recommend strains (cartridges) based on user behavior (e.g., 'We noticed that you are mostly using your vaporizer at night. Other people who use at night prefer this strain.').

The vaporizer or app may also include access to forums or chat areas where users may trade tips, and areas where physicians can discuss various topics.

In general, any of these apparatuses may permit users to engage in competition either by incentivizing usage or by including competitions that may be entered into by users (including multiple users) unrelated to vaporization of material. For example, gamification of usage (including purchasing of new components such as cartridges) may include awarding points, prizes, etc. and the creation of teams for switching or the like. Competitions may include the use of the accelerometer or other sensors in the apparatus that may be transmitted wirelessly to an app and/or to another user's vaporizer or app (e.g., directly or via a remote server) to permit competition interaction.

The vaporizers and/or apps described herein may also facilitate sponsorships, for example, allowing a user to sign a friend or family member up, pay the cost for a vaporizer, and have it sent to them or even delivered immediately (e.g., by bike messenger). This may be used to provide incentives with sponsors for switching from traditional cigarettes to vaporizers and/or reward use (presumably in place of use of traditional cigarettes), e.g., if you stick with it you get prizes (e.g., gift cards, etc.).

Any of the apparatuses described herein (including the vaporizers and any affiliated apps) may also be used to collect and analyze user data. This may allow the vaporizer producers, providers and retailers to get to know users better, including understand where when and how they are using the vaporizer. Knowing where and when a consumer is using a vaporizer may allow better marketing to users and may improve the design for future products.

The vaporizers and apps described herein may also facilitate communication between the manufacturer and/or retailer and the consumer (user). For example, by interacting with consumers while they are using the product, there may be opportunities to encourage direct sales. Thus, for example an app may say: "If my calculations are correct, it looks like you only have one cartridge left in your pack. Would you like to buy another?"

The vaporizers and apps described herein may also have enhanced anti-counterfeit components, including registration (e.g., through use of the app) of the vaporizer and/or app. In some implementations, the vaporizer could have a similar encryption handshake with the app and/or the charging dock.

In addition, the vaporizers and/or the app may permit or include device diagnostics. For example, the vaporizer and/or app may monitor component level failures (e.g., pressure sensor, battery, pogo pins, etc.), and may potentially identify a broken device in the field and ship warranty replacement without the need to return device to customer service. This may also permit the faster collection of data on common problems to be used for rolling changes and future designs.

### EXAMPLE: Application software/hardware/firmware ("App")

**VAPORIZABLE MATERIAL.** As described above, a vaporizer and/or vaporizer system consistent with implementations of the current subject matter may be used with (and may include or be configured specifically for) any appropriate vaporizable material. In certain implementations, the vaporizable material is an organic material. In certain examples, vaporizable material includes a liquid, a viscous liquid, a wax, a loose-leaf plant material, etc. In certain examples, the vaporizable material is a tobacco-based material. In certain examples, the vaporizable material is a botanical. In certain examples, the vaporizable material is nicotine, a nicotine derivative or a nicotine salt. In certain examples, the vaporizable material is a nutraceutical. In certain examples, the vaporizable material contains a cannabinoid. In certain examples, the vaporizable material is a medicinal compound.

In certain examples, the vaporizable material exhibits a viscosity between 1 and 50 Centipoise. In certain implementations, the vaporizable material exhibits a viscosity between 50 and 1,000 Centipoise. In certain examples, the vaporizable material exhibits a viscosity between 1,000 and 5,000 Centipoise. In certain examples, the vaporizable material exhibits a viscosity between 5,000 and 10,000 Centipoise. In certain examples, the vaporizable material exhibits a viscosity above 10,000 Centipoise.

In certain examples, the vaporizable material contains nicotine. In certain examples, the vaporizable material contains a nicotine derivative. In certain examples, the nicotine derivative is an acid salt of nicotine. In certain implementations, the acid salt of nicotine comprises an organic acid. In certain examples, the acid salt of nicotine does not comprise an inorganic acid.

In certain examples, the vaporizable material is a formulation of nicotine, nicotine derivatives, or a nicotine salt. In some formulations the concentration of nicotine or derivatives thereof in the formulation is about 1% (w/w) to about 25% (w/w). In some formulations the concentration of nicotine or derivatives thereof; in the formulation is about 1% (w/w) to about 20% (w/w). In some formulations the concentration of nicotine in the formulation is about 1% (w/w) to about 18% (w/w). In some examples, the concentration of nicotine in the formulation is about 1% (w/w) to about 15% (w/w). In some examples, the concentration of nicotine in the formulation is about 1% (w/w) to about 10% (w/w). In some examples, the concentration of nicotine in the formulation is about 1% (w/w) to about 8% (w/w). In some examples, the concentration of nicotine in the formulation is about 2% (w/w) to about 10% (w/w). In some formulations the concentration of nicotine in the formulation is about 4% (w/w) to about 12% (w/w). In some formulations the concentration of nicotine in the formulation is about 4% (w/w). In some examples, the concentration of nicotine in the formulation is about 2% (w/w).

Nicotine salt formulations are formed by the addition of a suitable acid to nicotine or a derivative thereof, including organic or inorganic acids. In some formulations provided herein, suitable organic acids are carboxylic acids. Examples of organic carboxylic acids disclosed herein are monocarboxylic acids, dicarboxylic acids (organic acid containing two carboxylic acid groups), carboxylic acids containing an aromatic group such as benzoic acids, hydroxycarboxylic acids, heterocyclic carboxylic acids, terpenoid acids, sugar acids; such as the pectic acids, amino acids, cycloaliphatic acids, aliphatic carboxylic acids, keto carboxylic acids, and the like. In some formulations provided herein, the organic acids used herein are monocarboxylic acids. In some formulations provided herein the organic carboxylic acid is benzoic, levulinic, acetic, lactic, citric, sorbic, lauric, salicylic, pyruvic or a combination thereof. In some formulations provided herein the organic carboxylic acid is not levulinic. Nicotine salts are formed from the addition of a suitable acid to nicotine. In some formulations provided herein, the stoichiometric ratios of the nicotine to acid (nicotine: acid) are 1:1, 1:2, 1:3, 1:4, 2:3, 2:5, 2:7, 3:4, 3:5, 3:7, 3:8, 3:10, 3:11, 4:5, 4:7, 4:9, 4:10, 4:11, 4:13, 4:14, 4:15, 5:6, 5:7, 5:8, 5:9, 5:11, 5:12, 5:13, 5:14, 5:16, 5:17, 5:18, or 5:19. In some formulations provided herein, the stoichiometric ratios of the nicotine to acid are 1:1, 1:2, 1:3, or 1:4 (nicotine: acid).

In certain examples, the pH of the nicotine formulation is acidic. In certain examples, the pH of the nicotine formulation is < 7.0. In certain examples, the pH of the nicotine formulation is < 6.0. In certain examples, the pH of the nicotine formulation is < 5.0. In certain examples, the pH of the nicotine formulation is < 4.0. In certain examples, the pH of the nicotine formulation is >3.0. In certain examples, the pH of the nicotine formulation is >4.0. In certain examples, the pH of the nicotine formulation is >5.0. In certain examples, the pH of the nicotine formulation is >6.0.

In certain examples, the vaporizable material contains a medicinal compound as an active ingredient. The medicinal compounds that are active ingredients for vaporization with the electronic vaporizer device utilizing the method herein, include drugs that can be heated without combustion to vaporization for inhalation delivery at a temperature range of, e.g., about 100 °C (e.g., for water-based carriers, e.g., about 100 °C , 105°C, 110°C, 120°C, 130°C, 140°C, 150°C, 160°C, 170°C, etc.; for ethanol-based formulations, e.g., about 50°C, about 60°C, about 70°C, about 80°C, etc.) to about (e.g., below) the temperature at which the active ingredient thermally decomposes (e.g., less than about 150°C, 160°C, 170°C, 180°C, 190°C, 200°C, 210°C, 220°C, 230°C, 240°C, 250°C, 260°C, 270°C, 280°C, 290°C, 300°C, etc.). In certain examples, the drugs can be neat or are solubilized in a pharmaceutically acceptable solvent. In certain examples, the drugs can include over the counter (OTC) substances as aides for various ailments; wherein said drugs can include known respiratory aides for asthma or chronic obstructive pulmonary disease (COPD). The vaporizable materials that are active ingredients for vaporization with the device(s) herein described, can include drugs that can be heated to vaporization for inhalation delivery, without combustion; wherein said drugs can include over the counter (OTC) substances from the group comprising upper respiratory aides (like cetirizine), analgesics and internal medication aides (like ibuprofen, naproxen), heartburn aides (like omeprazole), sleeping aides (like doxylamine, diphenhydramine, melatonin), or motion sickness aides (like meclizine). In certain examples, the vaporizable material can contain respiratory aides for asthma or chronic obstructive pulmonary disease (COPD) such as short acting beta-agonist (like albuterol, levalbuterol, pirbuterol), long acting beta-agonist (like salmeterol, formoterol), anti-cholinergics (like atropine sulfate, ipratropium bromide), leukotriene modifiers (like montelukast, zafirlukast), cartico-steriods (like fluticasone, budesonide, mometasone), theophylline (like theophylline), or combination corticosteroid and beta agonist, long lasting (fluticasone and salmeterol, budesonide and formoterol, mometasone and formoterol). In certain examples, the vaporizable material can contain botanicals and/or nutraceuticals such as tea (polyphenols, flavonoids, green tea catechins +/- caffeine); horehound (phenol flavonoid glycosides, labdane diterpenoids, yohimbe, cranberry/grape(proanthocyanidins), black cohosh (terpene glycoside fraction (actine/cimifugoside), flax seed (omega fatty acids), echinacea (echinacoside), valerian (alkaloids, gabapentin, isovaleric acid, terpenes), senna (senna cglycosides), cinnamon (cinnamaldehyde, phenols, terpenes), vitamin D, saw palmetto (fatty acids), or caffeine. In certain examples, the vaporizable material is soluble to at least fifty percent by weight in any suitable carrier solvent such as glycols (such as propylene glycol and vegetable glycerin), ethylene glycol, dipropylene glycol, trimethylene glycol, ethanol, and combinations thereof. In certain examples, the medicinal compound is terpinolene. In certain examples, the medicinal compound is Linalool. In certain examples, the medicinal compound is phytol. In certain examples, the medicinal compound is beta myrcene. In certain examples, the medicinal compound is citronellol. In certain examples, the medicinal compound is caryophyllene oxide. In certain examples, the medicinal compound is alpha pinene. In certain examples, the medicinal compound is limonene. In certain examples, the medicinal compound is beta caryophyllene. In certain examples, the medicinal compound is humulene. In certain implementations, the vaporizable material is an essential oil.

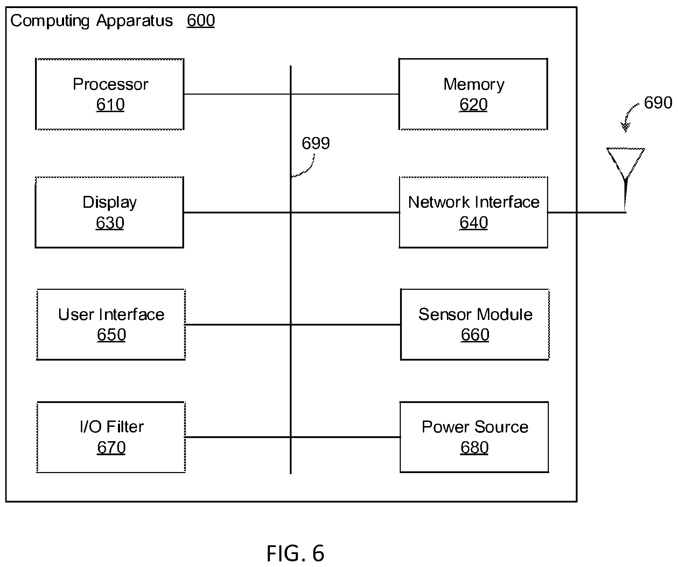

FIG. 6 illustrates an example computing apparatus 600 which may be used to implement one or more of the described features and/or components, in accordance with some example implementations. For example, at least a portion of the computing apparatus 600 may be used to implement at least a portion of the vaporizer 100, the vaporizer 200, the user device 305, the remote server 307, and/or like. The components of the computing apparatus 600 can be implemented in addition to or alternatively from any of the components of the vaporizer apparatuses 100, 200 illustrated and/or described. Computing apparatus 600 may be implemented to perform one or more of the processes described herein.

The computing apparatus 600 may perform one or more of the processes described herein. For example, the computing apparatus 600 may be used to execute an application providing for user control of a vaporizer in communication with the computing apparatus 600 and/or to provide an interface for the user to engage and interact with functions related to the vaporizer, in accordance with some example implementations.

As illustrated, the computing apparatus 600 may include one or more processors such as processor 610 to execute instructions that may implement operations consistent with those described herein. The computing apparatus 600 may include memory 620 to store executable instructions and/or information. Memory 620 may include solid-state memory, solid-state disk drives, magnetic disk drives, or any other information storage device. The computing apparatus 600 may include a network interface 640 to a wired network or a wireless network, such as the network described with reference to FIG. 5. In order to effectuate wireless communications, the network interface 640, for example, may utilize one or more antennas, such as antenna 690.

The computing apparatus 600 may include one or more user interfaces, such as user interface 650. The user interface 650 can include hardware or software interfaces, such as a keyboard, mouse, or other interface, some of which may include a touchscreen integrated with a display 630. The display 630 may be used to display information, such as information related to the functions of a vaporizer, provide prompts to a user, receive user input, and/or the like. In various implementations, the user interface 650 can include one or more peripheral devices and/or the user interface 650 may be configured to communicate with these peripheral devices.

In some aspects, the user interface 650 may include one or more sensors and/or may include an interface to one or more sensors, such as those described herein. The operation of these sensors may be controlled, at least in part, by a sensor module 660. The computing apparatus 600 may comprise an input and output filter 670, which can filter information received from the sensors or other user interfaces, received and/or transmitted via the network interface 640, and/or the like. For example, signals detected through the sensors can be passed through the filter 670 for proper signal conditioning, and the filtered data may then be passed to the sensor module 660 and/or processor 610 for validation and processing (e.g., before transmitting results or an indication via the network interface 640). The computing apparatus 600 may be powered through the use of one or more power sources, such as power source 680. As illustrated, one or more of the components of the computing apparatus 600 may communicate and/or receive power through a system bus 699.

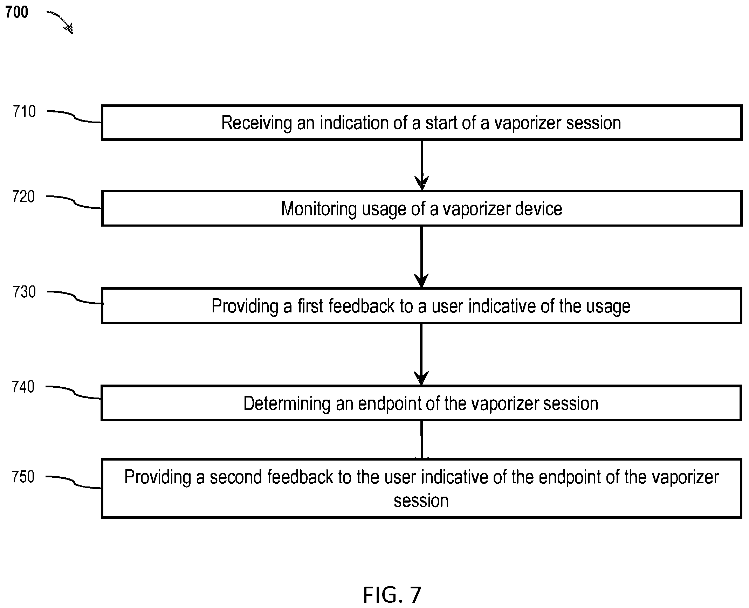

As noted above, implementations of the current subject matter include various methods of use of vaporizers and vaporizer systems that include a device in communication with a vaporizer. FIG. 7 illustrates a flowchart of a method 700 for providing sessioning information to a user, in accordance with some example implementations. In various implementations, the method 700 (or at least a portion thereof) may be performed by one or more of the vaporizer 100, the vaporizer 200, the user access device 305, the remote server 307, the computing apparatus 600, other related apparatuses, and/or some portion thereof.

Method 700 can start at operational block 710 where the apparatus 600, for example, can receive an indication of a start of a vaporizer session. Method 700 can proceed to operational block 720 where the apparatus 600, for example, can monitor usage of a vaporizer device. In some aspects, the apparatus 600 monitors usage by receiving data from one or more sensors associated with the apparatus 600. Method 700 can proceed to operational block 730 where the apparatus 600, for example, can provide a first feedback to a user indicative of the usage. Method 700 can proceed to operational block 740 where the apparatus 600, for example, can determine an endpoint of the vaporizer session. Method 700 can proceed to operational block 750 where the apparatus 600, for example, can provide a second feedback to the user indicative of the endpoint of the vaporizer session.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present.

Although described or shown with respect to a given example, the features and elements so described or shown can apply to other implementations of the current subject matter. It will also be appreciated by those of skill in the art that references to a structure or feature that is disposed "adjacent" another feature may have portions that overlap or underlie the adjacent feature.

Terminology used herein is for the purpose of describing particular implementations and implementations only and is not intended to be limiting. For example, as used herein, the singular forms "a", "an" and "the" are intended to include the plural forms as well, unless the context clearly indicates otherwise. It will be further understood that the terms "comprises" and/or "comprising," when used in this specification and in the claims, specify the presence of stated features, steps, operations, elements, and/or components, but do not preclude the presence or addition of one or more other features, steps, operations, elements, components, and/or groups thereof.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings provided herein.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/-5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative implementations are described above, any of a number of changes may be made to various implementations without departing from the teachings herein. For example, the order in which various described method steps are performed may often be changed in alternative implementations, and in other alternative implementations one or more method steps may be skipped altogether. Optional features of various device and system implementations may be included in some implementations and not in others. Therefore, the foregoing description is provided primarily for exemplary purposes and should not be interpreted to limit the scope of the claims.

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input. Other possible input devices include, but are not limited to, touch screens or other touch-sensitive devices such as single or multi-point resistive or capacitive trackpads, voice recognition hardware and software, optical scanners, optical pointers, digital image capture devices and associated interpretation software, and the like

The examples and illustrations included herein show, by way of illustration and not of limitation, specific implementations in which the subject matter may be practiced. As mentioned, other implementations may be utilized and derived there from, such that structural and logical substitutions and changes may be made without departing from the scope of this disclosure. Such implementations of the inventive subject matter may be referred to herein individually or collectively by the term "invention" merely for convenience and without intending to voluntarily limit the scope of this application to any single invention or inventive concept, if more than one is, in fact, disclosed. Thus, although specific implementations have been illustrated and described herein, any arrangement calculated to achieve the same purpose may be substituted for the specific implementations shown. This disclosure is intended to cover any and all adaptations or variations of various implementations. Combinations of the above implementations, and other implementations not specifically described herein, will be apparent to those of skill in the art upon reviewing the above description.

### ASPECTS OF THE DISCLOSURE

The disclosure also relates to the following aspects of the invention:
1. A vaporizer system comprising:
   a vaporizer device having one or more sensors for sensing a usage of a vaporizable material in a period of time, the one or more sensors being configured to generate usage data representing the usage of the vaporizable material, the vaporizer device further having a first transceiver for communicating the usage data to a wireless channel; and
   a mobile communication device having a second transceiver configured for being in communication with the first transceiver of the vaporizer device via the wireless channel, the mobile communication device having a memory that stores an application, a processor for executing the application, and a user interface for displaying an output of the application, the application being configured to:
      receive, via the user interface, usage preferences of a preferred usage of the vaporizable material by a user of the vaporizer device;
      receive, via the second transceiver, the usage data representing the usage of the vaporizable device;
      compare the usage data with the usage preferences within a predetermined time period;
      determine a set of operational settings for the vaporizer device that correspond to the user's preferred usage of the vaporizable material; and
      transmit, via the second transceiver to the vaporizer device over the wireless channel, the set of operational settings for the vaporizer device.
2. The vaporizer system in accordance with aspect 1, wherein the usage preferences include an amount of nicotine drawn from the vaporizer device for the predetermined time period.
3. The vaporizer system in accordance with aspect 1, wherein the usage preferences include a dosage limit of the vaporizable material.
4. The vaporizer system in accordance with aspect 3, wherein the dosage limit is specified per use of the vaporizer device.
5. The vaporizer system in accordance with aspect 3, wherein the dosage limit is specified per the predetermined time period.
6. The vaporizer system in accordance with any of aspects 1 to 5, wherein the usage preferences are received by the vaporizer device.
7. The vaporizer system in accordance with any of aspects 1 to 6, wherein the usage preferences include one or more user actions required before the vaporizer device can be used.
8. The vaporizer system in accordance with aspect 7, wherein the one or more user actions include at least one of an input action with the application and a physical action with the vaporizer device.
9. A vaporizer device that is configured to communicate with an application being executed by a mobile computing device, the application being configured to receive usage preferences of preferred usage of a vaporizable material by a user of the vaporizer device, and to determine a set of operational settings for the vaporizer device that correspond to the user's preferred usage of the vaporizable material, the vaporizer device comprising:
   a vaporizer body having a power source, a receptacle, and a controller;
   a cartridge configured for mating with the receptacle of the vaporizer body, and having a reservoir to hold the vaporizable material and a heater for vaporizing the vaporizable material based on a usage of the vaporizer device by the user, the heater being responsive to the set of operational settings managed by the controller;
   one or more sensors associated with the vaporizer body and/or the cartridge for sensing the usage of a vaporizable material in a period of time, the one or more sensors being configured to generate usage data representing the usage of the vaporizable material; and
   a transceiver for communicating the usage data to the mobile computing device via a wireless channel, and for receiving the set of operational settings to control the vaporizer device according to the usage preferences of the preferred usage of the vaporizable material received from the user of the vaporizable device.
10. The vaporizer device in accordance with aspect 9, wherein the usage preferences include an amount of nicotine drawn from the vaporizer device for the period of time, , and wherein the set of operational settings to control the vaporizer include an operational setting to limit the amount of nicotine drawn from the vaporizer device.
11. The vaporizer device in accordance with aspect 9, wherein the usage preferences include a dosage limit of the vaporizable material, and wherein the set of operational settings to control the vaporizer include an operational setting to limit the dosage of the vaporizable material by the vaporizer device.
12. The vaporizer device in accordance with aspect 11, wherein the dosage limit is specified per use of the vaporizer device.
13. The vaporizer device in accordance with aspect 11, wherein the dosage limit is specified per the predetermined time period.
14. The vaporizer device in accordance with any of aspects 9 to 13, further comprising a user input device associated with the vaporizer body, the user input device for receiving the usage preferences.
15. The vaporizer device in accordance with any of claims 9 to 14, wherein the usage preferences include one or more user actions required before the vaporizer device can be used.
16. The vaporizer system in accordance with aspect 15, wherein the one or more user actions include at least one of an input action with the application and a physical action with the vaporizer device.
17. A method of operating a vaporizer device that contains a vaporizable material for being vaporized by a user, the vaporizer device being configured to communicate with an application that is executed by a mobile computing device having a user interface, the method comprising:
   receiving, by the application, usage preferences of a preferred usage of the vaporizable material by the user of the vaporizer device;
   receiving, by the application from one or more sensors associated with the vaporizer device, usage data representing a usage of the vaporizable device;
   comparing, by the application, the usage data with the usage preferences within a predetermined time period;
   determining, by the application, a set of operational settings for the vaporizer device that correspond to the user's preferred usage of the vaporizable material; and
   transmitting, by the mobile computing device to the vaporizer device over a wireless channel, the set of operational settings for the vaporizer device to control an operation of the vaporizer device.
18. The method in accordance with aspect 17, wherein the usage preferences include an amount of nicotine drawn from the vaporizer device for the predetermined time period.
19. The method in accordance with aspect 17, wherein the usage preferences include a dosage limit of the vaporizable material.
20. The method in accordance with aspect 19, wherein the dosage limit is specified per use of the vaporizer device.
21. The method in accordance with aspect 19, wherein the dosage limit is specified per the predetermined time period.
22. The method in accordance with any of aspects 17 to 21, wherein the usage preferences are received by one or more of the user interface of the mobile computing device and an input device of the vaporizer device.
23. The method in accordance with any of aspects 17 to 22, wherein the usage preferences include one or more user actions required before the vaporizer device can be used.
24. The method in accordance with aspect 23, wherein the one or more user actions include at least one of an input action with the application and a physical action with the vaporizer device.

## Claims

1. A vaporizer system comprising:
a vaporizer device having one or more sensors for sensing a usage of a vaporizable material in a period of time, the one or more sensors being configured to generate usage data representing the usage of the vaporizable material, the vaporizer device further having a first transceiver for communicating the usage data to a wireless channel; and
a mobile communication device having a second transceiver configured for being in communication with the first transceiver of the vaporizer device via the wireless channel, the mobile communication device having a memory that stores an application, a processor for executing the application, and a user interface for displaying an output of the application, the application being configured to:
receive, via the user interface, usage preferences of a preferred usage of the vaporizable material by a user of the vaporizer device;
receive, via the second transceiver, the usage data representing the usage of the vaporizable device;
compare the usage data with the usage preferences within a predetermined time period;
determine a set of operational settings for the vaporizer device that correspond to the user's preferred usage of the vaporizable material; and
transmit, via the second transceiver to the vaporizer device over the wireless channel, the set of operational settings for the vaporizer device.

2. The vaporizer system in accordance with claim 1, wherein the usage preferences include an amount of nicotine drawn from the vaporizer device for the predetermined time period.

3. The vaporizer system in accordance with claim 1, wherein the usage preferences include a dosage limit of the vaporizable material.

4. The vaporizer system in accordance with claim 3, wherein the dosage limit is specified per use of the vaporizer device.

5. The vaporizer system in accordance with claim 3, wherein the dosage limit is specified per the predetermined time period.

6. The vaporizer system in accordance with any of claims 1 to 5, wherein the usage preferences are received by the vaporizer device.

7. The vaporizer system in accordance with any of claims 1 to 6, wherein the usage preferences include one or more user actions required before the vaporizer device can be used.

8. The vaporizer system in accordance with claim 7, wherein the one or more user actions include at least one of an input action with the application and a physical action with the vaporizer device.

9. The vaporizer system in accordance with any of claims 1 to 8, wherein the application is configured to provide a suggested dosage limit per period of time.

10. The vaporizer system in accordance with claim 9, wherein the suggested dosage limit is based on user data provided through the user interface.

11. The vaporizer system in accordance with claim 10, wherein the user data comprises a previous cigarette and/or vaping consumption over a set period of time.

12. The vaporizer system in accordance with any of claims 1 to 11, wherein the vaporizer device is configured to lock the user out for a sufficient period of time to allow the user to experience a full session during the next use of the vaporizer device and notify the user when a target has been exceeded.

13. The vaporizer device in accordance with any of claims 1 to 12, wherein the usage preferences include an amount of nicotine drawn from the vaporizer device for the period of time, and wherein the set of operational settings to control the vaporizer include an operational setting to limit the amount of nicotine drawn from the vaporizer device.

14. The vaporizer device in accordance with any of claims 1 to 12, wherein the usage preferences include a dosage limit of the vaporizable material, and wherein the set of operational settings to control the vaporizer include an operational setting to limit the dosage of the vaporizable material by the vaporizer device.
